# EUROPEAN PATENT SPECIFICATION

(11) **EP 4 313 987 B1**
(45) Date of publication and mention of the grant of the patent: **14.01.2026**
(21) Application number: 22719896.7
(22) Date of filing: 31.03.2022
(51) Int. Cl.: C07D 487/04

(54) **PROCESS FOR THE PREPARATION OF RIBOCICLIB AND PHARMACEUTICALLY ACCEPTABLE SALTS THEREOF**
VERFAHREN ZUR HERSTELLUNG VON RIBOCICLIB UND PHARMAZEUTISCH AKZEPTABLEN SALZEN DAVON
PROCÉDÉS DE PRÉPARATION DE RIBOCICLIB ET SES SELS PHARMACEUTIQUEMENT ACCEPTABLES

(30) Priority: 01.04.2021 EP 21166745; 25.02.2022 EP 22158967
(43) Date of publication of application: 07.02.2024
(73) Proprietor: KRKA, d.d., Novo mesto, 8000 Novo mesto (SI)
(72) Inventor: STANFEL, Ursa, 8000 Novo mesto (SI); BEZENSEK, Jure, 8000 Novo mesto (SI); ZUPANCIC, Katja, 8000 Novo mesto (SI); ROBEK, Spela, 8000 Novo mesto (SI); BERGANT SIMONCIC, Ana, 8000 Novo mesto (SI)
(74) Representative: Kutzenberger Wolff & Partner
(86) International application number: PCT/EP2022/058565
(87) International publication number: WO 2022/207788

(56) References cited:
- EP-A1- 3 231 805
- WO-A1-2018/051280
- WO-A1-2019/040567
- WO-A1-2019/142206
- WO-A1-2019/150181
- WO-A1-2020/084389
- WO-A1-2020/222256
- US-A1- 2019 345 163

## Description

Priority is claimed of European patent application no. 21 166 745.6 which was filed on April 1, 2021, and of European patent application no. 22 158 967.4 which was filed on February 25, 2022.

The invention relates to the synthesis of Ribociclib, and in particular to the synthesis of a 4-[6-[[7-cyclopentyl-6-(dimethylcarbamoyl)pyrrolo[2,3-d]pyrimidin-2-yl]amino]-3-pyridyl]-piperazine-1-carboxylate, which is an important intermediate in the synthesis of Ribociclib. Further, the invention relates to novel physiologically acceptable salts of Ribociclib.

Ribociclib (Kisqali^{®}) was approved by FDA and EMA in 2017 and is a cyclin-dependent kinase 4/6 inhibitor (CDK4/6) and is used for the treatment of certain kinds of breast cancer. Ribociclib has the systematic name 7-cyclopentyl-N,N-dimethyl-2-[[5-(1-piperazinyl)-2-pyridinyl]amino]-7H-pyrrolo-[2,3-d]pyrimidine-6-carboxamide (CAS Number: 1211441-98-3).

Various salts of Ribociclib have been described in e.g. WO 2010/020675 A1, WO 2016/091221 A1, WO 2018/051280 A1, WO 2019/040567 A1, WO 2019/062854 A1, WO 2019/082143 A1, WO 2020/222256 A1, CN 108 245 486 A, and CN 109 400 612 A.

Various synthetic concepts for the preparation of Ribociclib involving various building blocks.

One of these synthetic concepts involves synthesis of the intermediate tert-butyl 4-[6-[[7-cyclopentyl-6-(dimethylcarbamoyl)pyrrolo[2,3-d]pyrimidin-2-yl]amino]-3-pyridyl]piperazine-1-carboxylate 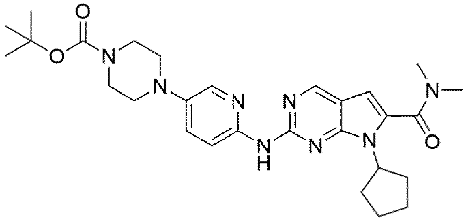 by reacting 2-chloro-7-cyclopentyl-7H-pyrrolo[2,3-d]pyrimidine-6-carboxylic acid dimethylamide (also referred to as 2-chloro-7-cyclopentyl-N,N-dimethyl-7H-pyrrolo[2,3-d]pyrimidine-6-carboxamide or 2-chloro-7-cyclopentyl-N,N-dimethyl-7H-pyrrolo[2,3-d]pyrimidine-6-carbamide) 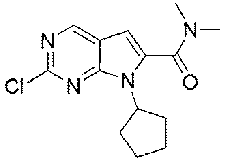 with tert-butyl 4-(6-aminopyridin-3-yl)piperazine-1-carboxylate (also referred to as 4-(6-aminopyridin-3-yl)piperazine-1-carboxylic acid tert-butyl ester)

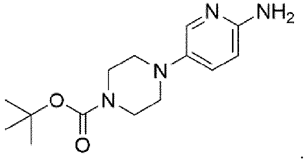

Deblocking the Boc-protective group from the above intermediate tert-butyl 4-[6-[[7-cyclopentyl-6-(dimethylcarbamoyl)pyrrolo[2,3-d]pyrimidin-2-yl]amino]-3-pyridyl]piperazine-1-carboxylate provides Ribociclib:

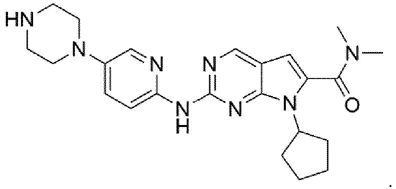

This reaction sequence is principally known from WO 2012/064805 A1, WO 2019/082143 A1, WO 2019/142206 A1, WO 2019/150181 A1, CN 106 749 259 A, CN 108 586 356 A, CN 109 400 612 A, and US 2018/16054443 A, US 2019/0345163 A1.

Other synthetic concepts that involve different building blocks and different intermediates are known from e.g. WO 2007/140222 A2, WO 2010/020675 A1, WO 2018/051280 A1, WO 2020/084389 A1, WO 2020/222256 A1.

The known concepts for coupling 2-chloro-7-cyclopentyl-7H-pyrrolo[2,3-d]pyrimidine-6-carboxylic acid dimethylamide and tert-butyl 4-(6-aminopyridin-3-yl)piperazine-1-carboxylate follow three different reaction types:
(i) copper-catalyzed Ullmann-Goldberg amination reactions (e.g. WO 2019/082143 A1);
(ii) palladium-catalyzed Buchwald-Hartwig amination reactions (e.g. WO 2012/064805 A1, CN 108 586 356 A, CN 109 400 612 A); and
(iii) aromatic nucleophilic substitution (e.g. US 2018/16054443 A, WO 2019/142206 A1, WO 2019/150181 A1). CN 106 749 259 A also relates to (iii) aromatic nucleophilic substitution, but the leaving group is a sulfonyl group instead of chlorine. US 2019/0345163 A1 also relates to (iii) aromatic nucleophilic substitution, but the leaving group and the amino group are exchanged.

As far as (i) Ullmann-Goldberg amination reactions are concerned in general, it is known that the need for stoichiometric amounts of copper and high reaction temperatures combined with the modest yields of product in most cases generally limits the application of these reactions. Despite these limitations, Ullmann-Goldberg amination reactions represented the state-of-the-art in metal-mediated C-N formation for nearly a century (see e.g. K.H. Shaughhnessy et al., Copper-Catalyzed Amination of Aryl and Alkenyl Electrophiles, Wiley, 2017). With respect to the synthesis of Ribociclib it would be desirable to avoid any heavy metals during synthesis and to improve reaction yields under moderate reaction conditions.

As far as (ii) palladium-catalyzed Buchwald-Hartwig amination reactions are concerned in general, it is known that they can be carried out under mild conditions with less reactive substrates. This resulted in palladium displacing copper as the preferred metal for aryl amination reactions in the late 1990s. With respect to the synthesis of Ribociclib it would be desirable to avoid costly palladium reagents and ligands such as 2,2'-bis(diphenylphosphino)-1,1'-binaphthyl (BINAP). It has also been reported that separating residual palladium from the product can cause substantial problems (L. Pellegatti et al., J. Flow Chem. 2016, 6(3), 198-201).

As far as (iii) aromatic nucleophilic substitutions (S_{N}Ar) are concerned in general, they typically require electron withdrawing groups that activate the ring towards nucleophilic attack. However, with respect to the synthesis of Ribociclib and its Boc-protected intermediate, it has been reported that strong non-nucleophilic bases such as lithium hexamethyldisilazide (LiHMDS) are required in order to achieve satisfactory conversions.

L. Pellegatti et al., J. Flow Chem. 2016, 6(3), 198-201 report that in a set of screening experiments, a mixture of 2-chloro-7-cyclopentyl-7H-pyrrolo[2,3-d]pyrimidine-6-carboxylic acid dimethylamide and tert-butyl 4-(6-aminopyridin-3-yl)piperazine-1-carboxylate was treated with different acids and bases, and the conversion was determined by high-performance liquid chromatography (HPLC). Neither acidic (conc. HCl, AcOH) nor classical base-promoted conditions led to conversion to the desired intermediate Boc-protected Ribociclib. It is reported that bases such as Hunigs base or DBU failed to promote the desired reaction, whereas potassium tertiary butanolate (KOtBu) resulted in decomposition of starting material 2-chloro-7-cyclopentyl-7H-pyrrolo[2,3-d]pyrimidine-6-carboxylic acid dimethylamide:

| | Additives | Equiv. | Solvent | Conditions | Conversion |
|---|---|---|---|---|---|
| a | HCl (37%) | 2.00 | NMP | MW 150°C, 10 min | no conversion |
| b | AcOH | Excess | - | MW 150°C, 10 min | no conversion |
| c | Hunigs base | 2.00 | NMP | MW 150°C, 10 min | no conversion |
| d | KOtBu | 2.00 | tBuOH | MW 100°C, 2 min | decomposition |
| e | DBU | 2.00 | tBuOH | MW 150°C, 2 min | no conversion |
| f | LiHMDS | 1.05 | THF | r.t., 40 min | 50% |
| g | LiHMDS | 2.20 | THF | r.t. 50 min | 94% |

However, by using more basic non-nucleophilic lithium hexamethyldisilazide (LiHMDS), L. Pellegatti et al., J. Flow Chem. 2016, 6(3), 198-201 report that satisfactory yields of 93% could be achieved using 2.2 equivalents of LiHMDS in tetrahydrofuran (THF) at room temperature. However, LiHMDS and its Na and K homologues are rather expensive and it would be desirable to achieve satisfactory conversion rates and good yields with less cost extensive reagents.

The synthetic concepts of the prior art are thus not satisfactory in every respect and there is a demand for synthetic routes making Ribociclib and its Boc-protected intermediate available at lower costs in good yields and reasonable reaction times. In particular, there is a demand for synthetic routes providing the Boc-protected intermediate of Ribociclib in yields of more than 70% using less expensive reactants, especially less expensive bases, without requiring special safety measures.

Furthermore, the physiologically acceptable salts of Ribociclib that are known from the prior art are not satisfactory in every respect and there is a demand for physiologically acceptable salts of Ribociclib that have advantages compared to these known salts. Providing new salts and crystalline or amorphous forms thereof is an important aspect in pharmaceutical development, because the different solid forms affect the properties of the medicinal product such as thermodynamic stability, storage stability, solubility, bioavailability, tendency to form solvates, density, hygroscopicity, adhesiveness, electrical properties, mechanical properties (such as compressibility, friability, hardness, breaking strength, elasticity), optical properties (such as color, transparency, refraction), and the like.

It is an object of the invention to provide a process for preparing Ribociclib and its protected intermediates which process has advantages compared to the prior art. It is a further object of the invention to provide physiologically acceptable salts of Ribociclib having advantages compared to the prior art.

This object has been achieved by the subject-matter of the patent claims.

It has been surprisingly found that the decomposition reaction reported by L. Pellegatti et al., J. Flow Chem. 2016, 6(3), 198-201 can be avoided when employing the reagents in a specific order thereby allowing to use potassium tertiary butanolate (KOtBu) as the base. Without wishing to be bound to any scientific theory, it appears that the coupling reaction can proceed via an S_{N}Ar mechanism and does not require any heavy metals or costly reagents such as copper, palladium, BINAP and the like.

Further, it has been surprisingly found that satisfactory yields are obtained in toluene as a solvent under mild reaction conditions (e.g. only 60 °C) within satisfactory time. Decomposition of starting material 2-chloro-7-cyclopentyl-7H-pyrrolo[2,3-d]pyrimidine-6-carboxylic acid dimethylamide in the presence of KOtBu in toluene is more evident at high temperatures of e.g. 90°C. When the reaction in toluene is performed at 60°C, decomposition is much less significant, whereas further decreasing the temperature to e.g. 30°C further suppresses decomposition. Such decomposition is also a function of the solvent. Further, it has been surprisingly found that excellent yields are obtained in THF as a solvent at reduced temperature (e.g. 0 °C) within satisfactory time. However, when the reaction is performed in acetonitrile, more side products are formed, i.e. the reaction proceeds less selectively.

Moreover, it has been surprisingly found that excellent yields and purities can be achieved when the stoichiometric ratio (mole : mole) of the 4-(6-aminopyridin-3-yl)piperazine-1-carboxylate of formula (III) to the 7-cyclopentyl-N,N-dimethyl-7H-pyrrolo[2,3-d]pyrimidine-6-carboxamide of formula (II) is close to equimolar, e.g. only about 1.2 : 1.0, or even only about 1.05 : 1.00. This is particularly advantageous because none of the two reactants needs to be employed in substantial excess.

Still further, it has been surprisingly found that quenching the reaction mixture with water improves the process performance.

Further, it has been surprisingly found that it is not necessary to use strong non-nucleophilic bases such as LiHMDS and its Na and K homologues, which are expensive, require special safety measures, and tend to yield undesirable Si species as side products. Satisfactory yields, e.g. of more than 70%, can be achieved with weaker and less expensive bases such as KOtBu. Further, it has been found that the required amount of a base such as KOtBu is lower, e.g. less than 2.5 equivalents are needed. Thus, Ribociclib and its Boc-protected intermediate can be provided at lower costs and reasonable reaction times on industrial scale.

Yet further, it has been surprisingly found that omitting heavy metals also improves the properties of the final drug (i.e. Ribociclib) because it is known that when heavy metals are used in the synthesis, trace amounts thereof can sometimes still be detected in the final drug. As the synthetic concept according to the present invention does not require any heavy metals, the final drug will be essentially free of any heavy metals.

Furthermore, it has been surprisingly found that the synthetic concept according to the present invention provides products that can be easily isolated and purified without requiring time consuming and costly silica gel chromatography, which is anyway laborious to be performed on industrial scale. Instead of such chromatographic purifications, the present invention allows for simple isolation procedures such as extraction and filtration.

Moreover, it has been surprisingly found that novel physiologically acceptable salts of Ribociclib can be provided that can be obtained as intermediates or that can be useful in therapy as such.

Further, it has been surprisingly found that deprotecting (deblocking in step (g) of the process according to the invention) can preferably be achieved by direct work up without intermediate extraction and crystallization . Thus, it has been surprisingly found that after alkalization the deprotected product can directly be isolated from an aqueous medium, whereas conventional intermediate steps after alkalizing can be omitted, namely adding organic solvents, extracting and then crystallizing the deprotected product; adding organic solvents into the aqueous medium after the reaction, however, is contemplated in accordance with the invention. As the deblocking step of the synthesis according to the invention preferably remains in the aqueous medium, it is less expensive and more beneficial with respect to the environment.

Another advantage of the synthesis according to the invention preferably using KOtBu as a base is that also at this stage precious and other metals are avoided over the whole procedure and consequently there is no need for additional Ribociclib purification steps in order to remove such precious or other metals.

A first aspect of the invention relates to a process for preparing a 4-[6-[[7-cyclopentyl-6-(dimethylcarbamoyl)pyrrolo[2,3-d]pyrimidin-2-yl]amino]-3-pyridyl]piperazine-1-carboxylate of formula (I) 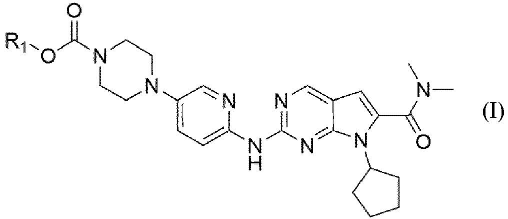 wherein R1 represents -C₁₋₁₂-alkyl, -C₁₋₁₂-aryl, or -C₁₋₁₂-alkyl-C₁₋₁₆-aryl; or -C₂₋₁₂-aryl, or -C₁₋₆-alkyl-C₂₋₁₆-aryl;
the process comprising the steps of:
(a) providing a composition comprising
   (i) a 4-(6-aminopyridin-3-yl)piperazine-1-carboxylate of formula (III) 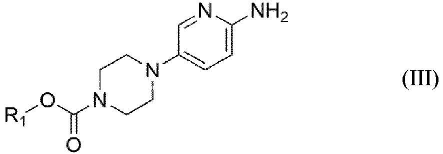 wherein R1 represents -C₁₋₁₂-alkyl, -C₁₋₁₂-aryl, or -C₁₋₁₂-alkyl-C₁₋₁₆-aryl; or -C₂₋₁₂-aryl, or - C₁₋₆-alkyl-C₂₋₁₆-aryl;
   (ii) a solvent which is selected from the group consisting of n-pentane, n-hexane, cyclohexane, methyl cyclohexane, toluene, benzene, xylene, dichloromethane, tetrahydrofuran (THF), and 1,4-dioxane; and
   (iii) a base;
      - wherein the conjugate acid of the base has a pKa value of at most 25; and
      - wherein the base has general formula (IV) 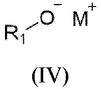 wherein M is selected from Li, Na, and K; and R₁ in general formula (IV) is selected from the group consisting of methyl, ethyl, -tert-butyl, -benzyl, -methylene-fluorenyl, and -allyl;
(b) adding to the composition provided in step (a) a 7-cyclopentyl-N,N-dimethyl-7H-pyrrolo[2,3-d]pyrimidine-6-carboxamide of formula (II) 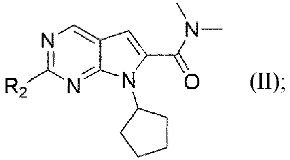 wherein R2 represents a leaving group;
(c) allowing the composition obtained in step (b) to react thereby obtaining a composition comprising the 4-[6-[[7-cyclopentyl-6-(dimethylcarbamoyl)pyrrolo[2,3-d]pyrimidin-2-yl]amino]-3-pyridyl]-piperazine-1-carboxylate of formula (I);
(d) optionally, adding an additional amount of base; preferably of the same base that was also contained in the composition provided in step (a);
(e) optionally, adding water to the composition obtained in step (c) or (d); and
(f) optionally, recovering the 4-[6-[[7-cyclopentyl-6-(dimethylcarbamoyl)pyrrolo[2,3-d]pyrimidin-2-yl]amino]-3-pyridyl]piperazine-1-carboxylate of formula (I) from the composition obtained in step (c), (d) or (e).

For the purpose of the specification, unless expressly stated otherwise, "essentially consisting of" preferably refers to a content of at least 95 wt.-%, more preferably at least 98 wt.-%, still more preferably at least 99.0 wt.-%, yet more preferably at least 99.90 wt.-%, and in particular 100 wt.-%.

For the purpose of the specification, unless expressly stated otherwise, all norms such as EN ISO, DIN or ASTM referred to herein, are referred to in the version that is valid on January 1, 2021.

Preferably, step (b) is performed at least 2.5 minutes after the composition has been provided in step (a); preferably at least 5 minutes, more preferably at least 7.5 minutes, still more preferably at least 10 minutes, yet more preferably at least 12.5 minutes, even more preferably at least 15 minutes, most preferably at least 17.5 minutes, and in particular at least 20 minutes.

Preferably, R1 in general formula (III) is selected from the group consisting of -methyl, -ethyl, -tert-butyl, -benzyl, -methylene-fluorenyl, and -allyl.

Preferably, R1 in general formula (III) is -tert-butyl.

Preferably, R2 in general formula (II) is selected from the group consisting of -Cl, -Br, -I, -O-S(-O)₂-CH₃, -O-S(=O)₂-CF₃, and -O-S(=O)₂-(C₆H₄)-CH₃.

Preferably, R2 in general formula (II) is -Cl.

Preferably, the reaction of the 4-(6-aminopyridin-3-yl)piperazine-1-carboxylate of formula (III) and the 7-cyclopentyl-N,N-dimethyl-7H-pyrrolo[2,3-d]pyrimidine-6-carboxamide of formula (II) in step (c) or (d) proceeds via an aromatic nucleophilic substitution (S_{N}Ar) mechanism.

Preferably, the composition provided in step (a) and/or the composition obtained in step (c) or (d) is a solution or a suspension.

In preferred embodiments, the stoichiometric ratio (mole : mole) of the 4-(6-aminopyridin-3-yl)piperazine-1-carboxylate of formula (III) to the a 7-cyclopentyl-N,N-dimethyl-7H-pyrrolo[2,3-d]pyrimidine-6-carboxamide of formula (II) is at least 0.5, or at least 0.6, or at least 0.7, or at least 0.8, or at least 0.9, or at least 1.0; more preferably at least 1.05 or at least 1.1, or at least 1.2, or at least 1.3, or at least 1.4, or at least 1.5, or at least 1.6, or at least 1.7, or at least 1.8, or at least 1.9, or at least 2.0.

In preferred embodiments, the stoichiometric ratio (mole : mole) of the 4-(6-aminopyridin-3-yl)piperazine-1-carboxylate of formula (III) to the a 7-cyclopentyl-N,N-dimethyl-7H-pyrrolo[2,3-d]pyrimidine-6-carboxamide of formula (II) is at most 2.5, or at most 2.4, or at most 2.3, or at most 2.2, or at most 2.1, or at most 2.0; more preferably at most 1.9, or at most 1.8, or at most 1.7, or at most 1.6, or at most 1.5, or at most 1.4, or at most 1.3, or at most 1.2, or at most 1.1, or at most 1.0.

In preferred embodiments, the stoichiometric ratio (mole : mole) of the 4-(6-aminopyridin-3-yl)piperazine-1-carboxylate of formula (III) to the 7-cyclopentyl-N,N-dimethyl-7H-pyrrolo[2,3-d]pyrimidine-6-carboxamide of formula (II) is within the range of from 1.5 : 1.0 to 1.0 : 1.5, more preferably 1.4 : 1.0 to 1.0 : 1.4, still more preferably 1.3 : 1.0 to 1.0 : 1.3, even more preferably 1.2 : 1.0 to 1.0 : 1.2, and most preferably 1.1 : 1.0 to 1.0 : 1.1.

In preferred embodiments, the stoichiometric ratio (mole : mole) of the 4-(6-aminopyridin-3-yl)piperazine-1-carboxylate of formula (III) to the 7-cyclopentyl-N,N-dimethyl-7H-pyrrolo[2,3-d]pyrimidine-6-carboxamide of formula (II) is within the range of 1.20±0.20 : 1.00, more preferably 1.20±0.15 : 1.00, still more preferably 1.20±0.10 : 1.00, and even more preferably 1.20±0.05 : 1.00.

In further preferred embodiments, the stoichiometric ratio (mole : mole) of the 4-(6-aminopy-ridin-3-yl)piperazine-1-carboxylate of formula (III) to the 7-cyclopentyl-N,N-dimethyl-7H-pyrrolo[2,3-d]pyrimidine-6-carboxamide of formula (II) is within the range of 1.05±0.05 : 1.00, more preferably 1.05±0.04 : 1.00, still more preferably 1.05±0.03 : 1.00, and even more preferably 1.05±0.02 : 1.00.

According to the invention, the base has general formula (IV) 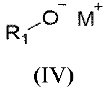 wherein M is selected from Li, Na, and K; preferably K.

Preferably, the base is an alkali metal alkoxide.

According to the invention, R1 in general formula (IV) is selected from the group consisting of -methyl, -ethyl, -tert-butyl, -benzyl, -methylene-fluorenyl, and -allyl.

Preferably, R1 in general formula (IV) is -tert-butyl.

Preferably, R1 in general formulas (I), (III) and (IV) has the same meaning, i.e. is identical.

Preferably, M in general formula (IV) is K.

Preferably, the base of general formula (IV) is potassium tert-butanolate (KOtBu).

In preferred embodiments, the conjugate acid of the base has a pKa value of
- at least 5.0, preferably at least 8.0, more preferably at least 10, still more preferably at least 12, yet more preferably at least 14, even more preferably at least 15, most preferably at least 16, and in particular at least 17;
- at most 20, and in particular at most 18; and/or
- within the range of 10±2.0, or 12±4.0, or 12±2.0, or 14±6.0, or 14±4.0, or 14±2.0, or 16±8.0, or 16±6.0, or 16±4.0, or 16±2.0, or 18±6.0, or 18±4.0, or 18±2.0, or 20±4.0, or 20±2.0, or 22±2.

In preferred embodiments, the stoichiometric ratio (mole : mole) of the base relative to the 7-cyclopentyl-N,N-dimethyl-7H-pyrrolo[2,3-d]pyrimidine-6-carboxamide of formula (II) is at least 0.5, or at least 0.6, or at least 0.7, or at least 0.8, or at least 0.9, or at least 1.0; more preferably at least 1.1, or at least 1.2, or at least 1.3, or at least 1.4, or at least 1.5, or at least 1.6, or at least 1.7, or at least 1.8, or at least 1.9, or at least 2.0.

In preferred embodiments, the stoichiometric ratio (mole : mole) of the base relative to the 7-cyclopentyl-N,N-dimethyl-7H-pyrrolo[2,3-d]pyrimidine-6-carboxamide of formula (II) is at most 10, or at most 9.0, or at most 8.0, or at most 7.0, or at most 6.0, or at most 5.0, or at most 4.9, or at most 4.8, or at most 4.7, or at most 4.6, or at most 4.5, or at most 4.4, or at most 4.3, or at most 4.2, or at most 4.1, or at most 4.0, or at most 3.9, or at most 3.8, or at most 3.7, or at most 3.6, or at most 3.5, or at most 3.4, or at most 3.3, or at most 3.2, or at most 3.1, or at most 3.0, or at most 2.9, or at most 2.8, or at most 2.7, or at most 2.6, or at most 2.5, or at most 2.4, or at most 2.3, or at most 2.2, or at most 2.1, or at most 2.0; more preferably at most 1.9, or at most 1.8, or at most 1.7, or at most 1.6, or at most 1.5, or at most 1.4, or at most 1.3, or at most 1.2, or at most 1.1, or at most 1.0.

In preferred embodiments, the stoichiometric ratio (mole : mole) of the base relative to the 7-cyclopentyl-N,N-dimethyl-7H-pyrrolo[2,3-d]pyrimidine-6-carboxamide of formula (II) is within the range of 1.6±0.25, or 1.65±0.5, or 1.65±0.25, or 1.7±0.75, or 1.7±0.5, or 1.7±0.25, or 1.75±1.0, or 1.75±0.75, or 1.75±0.5, or 1.75±0.25.

In preferred embodiments, the stoichiometric ratio (mole : mole) of the base relative to the 7-cyclopentyl-N,N-dimethyl-7H-pyrrolo[2,3-d]pyrimidine-6-carboxamide of formula (II) is within the range of 2.3±2.1 : 1.0, preferably 2.3±1.8 : 1.0, more preferably 2.3±1.5 : 1.0, still more preferably 2.3±1.2 : 1.0, yet more preferably 2.3±0.9 : 1.0, even more preferably 2.3±0.6 : 1.0, most preferably 2.3±0.3 : 1.0, and in particular 2.3±0.2 : 1.0.

In preferred embodiments, the stoichiometric ratio (mole : mole) of the base relative to the a 4-(6-aminopyridin-3-yl)piperazine-1-carboxylate of formula (III) is at least 0.5, or at least 0.6, or at least 0.7, or at least 0.8, or at least 0.9, or at least 1.0; more preferably at least 1.1, or at least 1.2, or at least 1.3, or at least 1.4, or at least 1.5, or at least 1.6, or at least 1.7, or at least 1.8, or at least 1.9, or at least 2.0.

In preferred embodiments, the stoichiometric ratio (mole : mole) of the base relative to the a 4-(6-aminopyridin-3-yl)piperazine-1-carboxylate of formula (III) is at most 2.5, or at most 2.4, or at most 2.3, or at most 2.2, or at most 2.1, or at most 2.0; more preferably at most 1.9, or at most 1.8, or at most 1.7, or at most 1.6, or at most 1.5, or at most 1.4, or at most 1.3, or at most 1.2, or at most 1.1, or at most 1.0.

In preferred embodiments, the base of general formula (IV), preferably potassium tert-butanolate (KOtBu), is added in pure form, i.e. as a solid.

In other preferred embodiments, the base of general formula (IV), preferably potassium tert-butanolate (KOtBu), is added in form of a solution in a suitable solvent.

In preferred embodiments, the solvent is the same as that provided in step (a) for the reaction of the 4-(6-aminopyridin-3-yl)piperazine-1-carboxylate of formula (III) with the 7-cyclopentyl-N,N-dimethyl-7H-pyrrolo[2,3-d]pyrimidine-6-carboxamide of formula (II). In other preferred embodiments, the solvent is different. For example, the reaction also proceeds under the same conditions in toluene and adding KOtBu solution in THF. Nonetheless, it has been found that using the same solvent can be advantageous with respect to performance.

When the base of general formula (IV), preferably potassium tert-butanolate (KOtBu), is added in form of a solution in a suitable solvent, the concentration of the base of general formula (IV) is preferably at least 5 wt.-%, more preferably at least 10 wt.-%, still more preferably within the range of from 10 to 25 wt.-%, in each case relative to the total weight of the solution.

. Preferably, at a later stage of the process according to the invention, the solvent additionally contains water that is added for work-up, i.e. after the reaction has taken place in subsequent steps. However, the solvent provided in step (a) is not being worked up and thus is essentially free of water.

According to the invention, the solvent is selected from the group consisting of n-pentane, n-hexane, cyclohexane, methyl cyclohexane, toluene, benzene, xylene, dichloromethane, tetrahydrofuran (THF), and 1,4-dioxane. Toluene and tetrahydrofuran (THF) are particularly preferred, tetrahydrofuran (THF) is most preferred.

Preferably, the solvent additionally contains water that is added for work-up, i.e. after the reaction has taken place.

In preferred embodiments, the solvent is a non-polar aprotic solvent selected from n-pentane, n-hexane, cyclohexane, methyl cyclohexane, toluene, benzene, xylene, tetrahydrofuran (THF), and 1,4-dioxane; preferably the solvent additionally contains water that is added for work-up, i.e. after the reaction has taken place.

In preferred embodiments, the solvent is a hydrocarbon solvent selected from n-pentane, n-hexane, cyclohexane, methyl cyclohexane, toluene, benzene, and xylene; preferably the solvent additionally contains water that is added for work-up, i.e. after the reaction has taken place.

In preferred embodiments, the solvent is toluene; preferably the solvent additionally contains water that is added for work-up, i.e. after the reaction has taken place.

In other preferred embodiments, the solvent is tetrahydrofuran (THF); preferably the solvent additionally contains water that is added for work-up, i.e. after the reaction has taken place.

In preferred embodiments, the solvent has a dipole moment of
- at least 0.025 D, preferably at least 0.05 D, more preferably at least 0.10 D, still more preferably at least 0.15 D, yet more preferably at least 0.20 D, even more preferably at least 0.25 D, most preferably at least 0.30 D, and in particular at least 0.35 D;
- at most 3.93 D, preferably at most 3.0 D, more preferably at most 1.78 D, still more preferably at most 1.7 D, yet more preferably at most 1.35 D, even more preferably at most 1.25 D, most preferably at most 0.50 D, and in particular at most 0.40 D; and/or
- within the range of 0.36±0.05 D, 0.55±0.20 D, 0.55±0.05 D, or 0.75±0.40 D, or 0.75±0.20 D, or 0.75±0.05 D or 1.0±0.65 D, or 1.0±0.40 D, or 1.0±0.20 D, or 1.0±0.05 D, or 1.25±0.90 D, or 1.25±0.65 D, or 1.25±0.40 D, or 1.25±0.20 D, or 1.25±0.05 D, or 1.50±1.15 D, or 1.50±0.90 D, or 1.50±0.0.65 D, or 1.50±0.40 D, or 1.50±0.20 D, or 1.50±0.05 D, or 1.75±1.40 D, 1.75±1.15 D, or 1.75±0.90 D, or 1.75±0.0.65 D, or 1.75±0.40 D, or 1.75±0.20 D, or 1.75±0.05 D, or 2.0±1.65 D, or 2.0±1.40 D, 2.0±1.15 D, or 2.0±0.90 D, or 2.0±0.0.65 D, or 2.0±0.40 D, or 2.0±0.20 D, or 2.0±0.05 D, or 2.25±1.90 D, or 2.25±1.65 D, or 2.25±1.40 D, 2.25±1.15 D, or 2.25±0.90 D, or 2.25±0.0.65 D, or 2.25±0.40 D, or 2.25±0.20 D, or 2.25±0.05 D.

In preferred embodiments, the solvent has a density at 20 °C of
- at least 0.62 g/cm³, preferably at least 0.67 g/cm³, more preferably at least 0.74 g/cm³, still more preferably at least 0.78 g/cm³, yet more preferably at least 0.79 g/cm³, even more preferably at least 0.80 g/cm³, most preferably at least 0.86 g/cm³, and in particular at least 0.89 g/cm³;
- at most 1.50 g/cm³, preferably at most 1.34 g/cm³, more preferably at most 1.18 g/cm³, still more preferably at most 1.02 g/cm³, yet more preferably at most 0.88 g/cm³, even more preferably at most 0.87 g/cm³, most preferably at most 0.80 g/cm³, and in particular at most 0.79 g/cm³; and/or
- within the range of 0.83±0.01 g/cm³, or 0.85±0.02 g/cm³, or 0.85±0.01 g/cm³, or 0.87±0.04 g/cm³, or 0.87±0.02 g/cm³, or 0.87±0.01 g/cm³, or 0.89±0.08 g/cm³, or 0.89±0.04 g/cm³, or 0.89±0.02 g/cm³, or 0.89±0.01 g/cm³, or 0.91±0.12 g/cm³, or 0.91±0.08 g/cm³, or 0.91±0.04 g/cm³, or 0.91±0.02 g/cm³, or 0.91±0.01 g/cm³, or 0.95±0.14 g/cm³, or 0.95±0.12 g/cm³, or 0.95±0.08 g/cm³, or 0.95±0.04 g/cm³, or 0.95±0.02 g/cm³, or 0.95±0.01 g/cm³;
in each case preferably determined according to ASTM 4052.

In preferred embodiments, the solvent has a boiling point of
- at least 35 °C, preferably at least 45 °C, more preferably at least 67 °C, still more preferably at least 75 °C, yet more preferably at least 82 °C, even more preferably at least 83 °C, most preferably at least 95 °C, and in particular at least 105 °C;
- at most 115 °C, preferably at most 103 °C, more preferably at most 85 °C, still more preferably at most 82 °C, yet more preferably at most 80 °C, even more preferably at most 70 °C, most preferably at most 67 °C, and in particular at most 45 °C; and/or
- within the range of 65±5.0 °C, or 70±10 °C, or 70±5.0 °C, or 85±15 °C, or 85±10 °C, or 85±5.0 °C, or 90±20 °C, or 90±15 °C, or 90±10 °C, or 90±5.0 °C, or 95±25 °C, or 95±20 °C, or -95±15 °C, or 95±10 °C, or 95±5.0 °C, or 100±30 °C, or 100±25 °C, or 100±20 °C, or 100±15 °C, or 100±10 °C, or 100±5.0 °C, or 105±35 °C, or 105±30 °C, or 105±25 °C, or 105±20 °C, or 105±15 °C, or 105±10 °C, or 105±5.0 °C, or 110±40 °C, or 110±35 °C, or 110±30 °C, or 110±25 °C, or 110±20 °C, or 110±15 °C, or 110±10 °C, or 110±5.0 °C;
in each case preferably determined according to ASTM D5399.

In preferred embodiments, step (a) comprises the sub-steps of:
(a-1) providing a composition comprising the 4-(6-aminopyridin-3-yl)piperazine-1-carboxylate of formula (III) and the solvent;
(a-2) optionally, cooling the composition provided in step (a-1); and
(a-3) adding the base to the composition provided in step (a-1).

In preferred embodiments, step (a-3) is performed at a temperature of
- at least -20 °C, preferably at least -15 °C, more preferably at least -10 °C, still more preferably at least -5.0 °C, yet more preferably at least 0 °C, even more preferably at least 5.0 °C, most preferably at least 10 °C, and in particular at least 15 °C;
- at most 25 °C, preferably at most 20 °C, more preferably at most 15 °C, still more preferably at most 10 °C, yet more preferably at most 5.0 °C, even more preferably at most 0 °C, most preferably at most -5 °C, and in particular at most -10 °C; and/or
- within the range of 15±5.0 °C, or 10±10 °C, or 10±5.0 °C, or 5.0±15 °C, or 5.0±10 °C, or 5.0±5.0 °C, or 0±20 °C, or 0±15 °C, or 0±10 °C, or 0±5.0 °C, or -5.0±25 °C, or -5.0±20 °C, or -5.0±15 °C, or -5.0±10 °C, or -5.0±5.0 °C.

In other preferred embodiments, preferably when the solvent is THF, step (a-3) is performed at a temperature of
- at least -20 °C, preferably at least -15 °C, more preferably at least -10 °C, still more preferably at least -5.0 °C;
- at most 20 °C, preferably at most 15 °C, more preferably at most 10 °C, still more preferably at most 5.0 °C; and/or
- within the range of 0±20 °C, or 0±15 °C, or 0±10 °C, or 0±5.0 °C.

In preferred embodiments, step (c) and/or (d) is performed at a temperature of
- at least 25 °C, preferably at least 30 °C, more preferably at least 35 °C, still more preferably at least 40 °C, yet more preferably at least 45 °C, even more preferably at least 50 °C, most preferably at least 55 °C, and in particular at least 60 °C; and/or
- at most 100 °C, preferably at most 95 °C, more preferably at most 90 °C, still more preferably at most 85 °C, yet more preferably at most 80 °C, even more preferably at most 75 °C, most preferably at most 70 °C, and in particular at most 65 °C; and/or
- at most 60 °C, preferably at most 55 °C, more preferably at most 50 °C, still more preferably at most 45 °C, yet more preferably at most 40 °C, even more preferably at most 35 °C, most preferably at most 30 °C, and in particular at most 25 °C; and/or
- within the range of 30±5.0 °C, or 35±10 °C, or 35±5.0 °C, or 40±15 °C, or 40±10 °C, or 40±5.0 °C, or 45±20 °C, or 45±15 °C, or 45±10 °C, or 45±5.0 °C, or 50±25 °C, or 50±20 °C, or 50±15 °C, or 50±10 °C, or 50±5.0 °C, or 55±30 °C, or 55±20 °C, or 55±15 °C, or 55±10 °C, or 55±5.0 °C, or 60±35 °C, or 60±30 °C, or 60±20 °C, or 60±15 °C, or 60±10 °C, or 60±5.0 °C, or 65±40 °C, or 65±35 °C, or 65±30 °C, or 65±20 °C, or 65±15 °C, or 65±10 °C, or 65±5.0 °C.

In other preferred embodiments, preferably when the solvent is THF or toluene, more preferably THF, step (c) and/or (d) is performed at a temperature of
- at least -20 °C, preferably at least -15 °C, more preferably at least -10 °C, still more preferably at least -5.0 °C;
- at most 20 °C, preferably at most 15 °C, more preferably at most 10 °C, still more preferably at most 5.0 °C; and/or
- within the range of 0±20 °C, or 0±15 °C, or 0±10 °C, or 0±5.0 °C.

In preferred embodiments, the adding of water in step (e) to the composition obtained in step (c) or (d) involves stirring and optionally subsequent stirring of the thus obtained aqueous composition. Preferably, subsequent stirring is performed for at least 15 minutes, more preferably at least 30 minutes. In preferred embodiments, subsequent stirring is performed at room temperature (about 23 °C). In other preferred embodiments, subsequent stirring is performed at elevated temperature, preferably within the range of from 30 to 60 °C.

In other preferred embodiments, the adding of water in step (e) to the composition obtained in step (c) or (d) involves direct removal of solvent, e.g. by evaporation. Experimental evidence indicates, however, that under these conditions purity of the desired product is slightly lower. Evaporation may optionally be performed under reduced pressure and/or at elevated temperature.

In preferred embodiments, the recovering in step (f) involves solvent extraction, filtration, trituration, washing, drying and/or recrystallization.

A preferred work-up procedure according to the invention ("*work-up procedure A*") includes
(i) removing of volatile compounds under vacuum;
(ii) dissolving the residue in a suitable organic solvent that is not (or only hardly) miscible with water, preferably dichloromethane, and washing the phase of the organic solvent with water or with an aqueous liquid;
(iii) separating the phase of the organic solvent from the aqueous phase;
(iv) concentrating the phase of the organic solvent, preferably under vacuum;
(v) optionally, triturating the thus obtained residue in a suitable solvent, preferably in methanol; and
(vi) optionally, filtering the thus triturated material; and
(vii) optionally, drying the thus filtered material to obtain the compound of formula (I).

Another preferred work-up procedure according to the invention ("*work-up procedure B*") includes
(i) removing of volatile compounds under vacuum;
(ii) triturating the thus obtained material;
(iii) filtering the thus obtained triturated material;
(iv) optionally, drying the thus filtered material;
(v) optionally, recrystallizing the thus obtained material;
(vi) filtering the thus obtained recrystallized material; and
(vii) optionally, drying the thus filtered material to obtain the compound of formula (I).

In preferred embodiments, the composition obtained in step (b) comprises, essentially consists of, or consists of
- the 4-(6-aminopyridin-3-yl)piperazine-1-carboxylate of formula (III);
- the solvent;
- the base; and
- the 7-cyclopentyl-N,N-dimethyl-7H-pyrrolo[2,3-d]pyrimidine-6-carboxamide of formula (II).

Preferably, the process according to the invention does not involve
- a copper-catalyzed amination reaction;
- a palladium catalyzed amination reaction; and
- an amination reaction involving an organosilicon base.

Preferably, the composition obtained in step (b) does not comprise
- copper (I) iodide, preferably no copper salt, more preferably no copper compound;
- palladium (II) acetate, [(2-di-cyclohexylphosphino-3,6-dimethoxy-2',4',6'-triisopropyl-1,1'-biphenyl)-2-(2'-amino-1,1'-biphenyl)]palladium (II) methanesulfonate (BrettPhos Pd G3), and tris(dibenzylideneacetone)dipalladium(0); preferably no palladium compound;
- 2,2'-bis(diphenylphosphino)-1,1'-binaphthyl) (BINAP), preferably no organophosphorus ligand;
- cesium carbonate, preferably no cesium base, more preferably no cesium compound; and/or
- lithium hexamethyldisilazide, sodium hexamethyldisilazide, and potassium hexamethyldisilazide; preferably no hexamethyldisilazide base, more preferably no organosilicon base, still more preferably no silicon compound.

Another aspect of the invention relates to a process for preparing Ribociclib 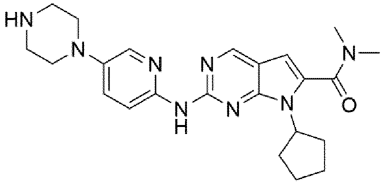 the process comprising a process according to the invention as described above and the additional step of:
(g) deblocking the 1N-amino group of the piperazine moiety of the 4-[6-[[7-cyclopentyl-6-(dimethylcarbamoyl)pyrrolo[2,3-d]pyrimidin-2-yl]amino]-3-pyridyl]piperazine-1-carboxylate of formula (I) 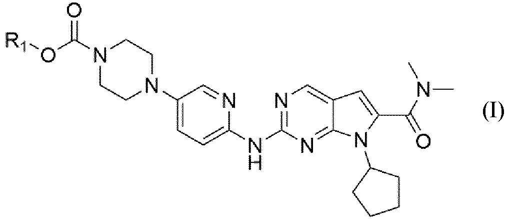 thereby obtaining Ribociclib.

Preferably, step (g) involves subjecting the 4-[6-[[7-cyclopentyl-6-(dimethylcarbamoyl)-pyrrolo[2,3-d]pyrimidin-2-yl]amino]-3-pyridyl]piperazine-1-carboxylate of formula (I) to acidic conditions.

Preferably, step (g) comprises the sub-steps of:
(g-1) contacting the 4-[6-[[7-cyclopentyl-6-(dimethylcarbamoyl)pyrrolo[2,3-d]pyrimidin-2-yl]amino]-3-pyridyl]piperazine-1-carboxylate of formula (I) with an acid thereby deblocking the 4-[6-[[7-cyclopentyl-6-(dimethylcarbamoyl)pyrrolo[2,3-d]pyrimidin-2-yl]amino]-3-pyridyl]piperazine-1-carboxylate of formula (I) and providing an acid addition salt of Ribociclib; and
(g-2) contacting the acid addition salt of Ribociclib with a base thereby providing Ribociclib.

In preferred embodiments, the acid is selected from the group consisting of hydrochloric acid, hydrobromic acid, formic acid, propionic acid, cinnamic acid, glutamic acid, glutaric acid, malonic acid, malic acid, and phthalic acid.

In preferred embodiments, the base used for alkalizing is selected from NaHCO₃, KHCO₃, Na₂CO₃, K₂CO₃, NaOH, KOH and KOtBu.

In preferred embodiments, step (g) of the process according to the invention, preferably sub-steps (g-1) and (g-2) are directly performed with the crude reaction product obtained in step (e) or (f), i.e. with the aqueous mixture or an isolated solid. Thus, the crude reaction product obtained in step (e) or (f) is preferably not worked-up prior to performing step (g).

In other preferred embodiments, step (f) of the process according to the invention involves solvent extraction, filtration, trituration, washing, drying and/or recrystallization; preferably according to *"work-up procedure A"* or according to *"work-up procedure B"* (see above); and step (g) of the process according to the invention, preferably sub-steps (g-1) and (g-2) are performed with the recrystallized reaction product obtained in step (f).

Another aspect of the invention relates to a process for preparing a physiologically acceptable salt of Ribociclib comprising a process for preparing Ribociclib as described above and the additional steps of:
(h) providing a composition comprising Ribociclib, an acid, a solvent A and optionally a solvent B;
(i) allowing the Ribociclib and the acid to form a physiologically acceptable salt of Ribociclib thereby providing a product composition; and
(j) optionally, recovering at least the majority of the physiologically acceptable salt of Ribociclib from the product composition.

Preferably, the composition provided in step (h) is a solution and/or a suspension.

In preferred embodiments, solvent A is selected from alcohols, ketones, esters, and nitriles.

In preferred embodiments, solvent A is selected from the group consisting of methanol, ethanol, n-propanol, isopropanol, n-butanol, isobutanol, tert-butanol, dichloromethane, acetone, butanone, ethyl acetate acetonitrile, and mixtures thereof; preferably isopropanol, methanol, and dichloromethane.

In preferred embodiments, solvent B is selected from alkanes, ethers, esters, alcohols and water.

In preferred embodiments, solvent B is selected from the group consisting of water, n-pentane, n-hexane, n-heptane, methanol, diethylether, tetrahydrofuran, 1,4-dioxane, tert-butylmethylether, ethyl acetate, and mixtures thereof; preferably water, methanol, and ethyl acetate.

In preferred embodiments, step (h) comprises the sub-steps of:
(h-1) providing a composition comprising Ribociclib, solvent A and optionally solvent B;
(h-2) heating the composition obtained in step (h-1) to reflux temperature;
(h-3) optionally, dissolving the acid in a solvent C and optionally a solvent D; and
(h-4) adding the acid or the optionally dissolved acid to the composition provided in step (h-2).

In preferred embodiments, solvent C is selected from alkanes, ethers, esters, alcohols and water.

In preferred embodiments, solvent C is selected from the group consisting of water, n-pentane, n-hexane, n-heptane, methanol, diethylether, tetrahydrofuran, 1,4-dioxane, tert-butylmethylether, ethyl acetate, and mixtures thereof; preferably water, methanol, and ethyl acetate.

In preferred embodiments, solvent D is selected from alcohols, ketones, esters, and nitriles.

In preferred embodiments, solvent D is selected from the group consisting of methanol, ethanol, n-propanol, isopropanol, n-butanol, isobutanol, tert-butanol, dichloromethane, acetone, butanone, ethyl acetate, acetonitrile, and mixtures thereof; preferably isopropanol, methanol, and dichloromethane.

In preferred embodiments, step (j) comprises the sub-steps of:
(j-1) cooling the product composition obtained in step (i); and/or
(j-2) adding solvent C and/or solvent D to the composition obtained in step (i).

Preferably, the acid is an organic acid.

Preferably, the acid is a carboxylic acid.

Another aspect of the invention relates to a physiologically acceptable salt of Ribociclib that is obtainable by the above process according to the invention.

In preferred embodiments, the acid is selected from the group consisting of hydrochloric acid, hydrobromic acid, sulfuric acid, phosphoric acid, methylsulfonic acid, 2-hydroxyethylsulfonic acid, benzenesulfonic acid, p-toluenesulfonic acid, carbonic acid, formic acid, acetic acid, glycolic acid, trifluoroacetic acid, propionic acid, 2-hydroxypropionic acid, oxalic acid, malonic acid, maleic acid, fumaric acid, malic acid, tartaric acid, glutaric acid, glutamic acid, citric acid, adipic acid, phthalic acid, mandelic acid, cinnamic acid; more preferably formic acid, propionic acid, cinnamic acid, glutamic acid, glutaric acid, malonic acid, malic acid, and phthalic acid.

In preferred embodiments the process comprises the following steps and sub-steps:
(h-1) providing a composition comprising Ribociclib, dichloromethane and methanol;
(h-2) heating the composition obtained in step (h-1) to an elevated temperature, preferably to about 30°C;
(h-3) adding the acid, preferably malonic acid, to the heated composition obtained in step (h-2);
(i) allowing the Ribociclib and the acid to form a physiologically acceptable salt of Ribociclib thereby providing a product composition;
(j-1) cooling the product composition obtained in step (i) to 23°C or below;
(j-2) adding further methanol to the cooled composition obtained in step (j-1) thereby inducing or completing precipitation of the physiologically acceptable salt of Ribociclib.

The following examples illustrate the invention and are not intended to restrict the scope of the invention in any way.

### Example 1 - Synthesis of tert-butyl 4-[6-[[7-cyclopentyl-6-(dimethylcarbamoyl)pyrrolo[2,3-d]pyrimi-din-2-yl]amino]-3-pyridyl]piperazine-1-carboxylate:

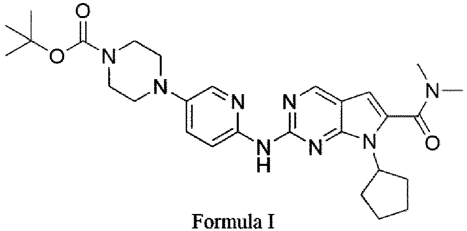

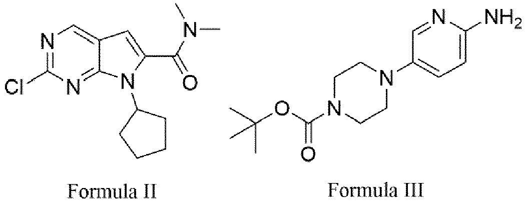

### Example 1-1:

To a cooled solution of tert-butyl 4-(6-aminopyridin-3-yl)piperazine-1-carboxylate of formula III (1.14 g, 1.2 eq.) in toluene (12 mL), potassium tert-butoxide (0.767 g; 2 eq.) was added. The resulting mixture was stirred for at least half an hour. To this mixture the compound 2-chloro-7-cyclopentyl-N,N-dimethyl-7H-pyrrolo[2,3-d]pyrimidine-6-carboxamide of formula II (1.00 g; 1.0 eq.) suspended in toluene (9 mL) was added and heated to 30±5 °C. The reaction mixture was stirred at this temperature for at least 3 hours.

Water (21 mL) was added and the mixture was stirred for at least 1 hour. The reaction mixture was further diluted with dichloromethane (30 mL) and 1% dissolution of acetic acid in water (20 mL). Layers were separated and the dichloromethane layer was washed with water (20 mL), separated and then concentrated under vacuum and the residue was triturated in methanol (6 mL), followed by filtration to obtain the compound of formula I (1.348 g; purity = 96.12 %; η = 70.95 %).

### Example 1-2:

A mixture of 3.42 g of tert-butyl 4-(6-aminopyridin-3-yl)piperazine-1-carboxylate of formula III (97 % NMR assay) in 36 mL THF was cooled to about 0 °C under inert atmosphere. 2.53 g of KOtBu in 11 mL THF were added and the resulting mixture was stirred at about 0 °C under inert atmosphere. The mixture was combined with 3.00 g of 2-chloro-7-cyclopentyl-N,N-dimethyl-7H-pyrrolo[2,3-d]pyrimidine-6-carboxamide of formula II (97 % NMR assay) in 27 mL THF at about 0 °C, and the resulting reaction mixture was stirred at about 0 °C under inert atmosphere for about 2 h.

Next, 63 mL of water were added at about 0 °C. After about 0.5-1 h of stirring at about 0 °C, the mixture was warmed to RT and stirred at RT for about 4 h. THF was then evaporated and the resulting mixture was stirred at RT for about 1 h. The mixture was filtered and the cake was washed with 30 mL of water. After drying overnight at RT, 5.195 g of crude product were obtained with 96.10 % HPLC purity.

3.000 g of the crude product were recrystallized from THF/MeOH to give 2.674 g tert-butyl 4-[6-[[7-cyclopentyl-6-(dimethylcarbamoyl)pyrrolo[2,3-d]pyrimidin-2-yl]amino]-3-pyridyl]piperazine-1-carboxylate of formula I with 99.43 % HPLC purity (85 % overall yield).

### Example 1-3:

A mixture of 6.84 g of tert-butyl 4-(6-aminopyridin-3-yl)piperazine-1-carboxylate of formula III (97 % NMR assay) in 72 mL THF was cooled to about 0 °C under inert atmosphere. 5.06 g of KOtBu in 23 mL THF were added and the resulting mixture was stirred at about 0 °C under inert atmosphere. The mixture was combined with 6.00 g of 2-chloro-7-cyclopentyl-N,N-dimethyl-7H-pyrrolo[2,3-d]pyrimidine-6-carboxamide of formula II (97 % NMR assay) in 54 mL THF at about 0 °C, and the resulting reaction mixture was stirred at about 0 °C under inert atmosphere for about 2 h.

Next, 126 mL of water were added at about 0 °C. The mixture was warmed to RT and stirred at RT overnight. THF was then evaporated and the resulting mixture was stirred at RT for about 1.5 h. The mixture was filtered and the cake was washed with 60 mL of water. After drying overnight at RT, 10.439 g of crude product were obtained with 93.42 % HPLC purity.

4.500 g of the crude product were recrystallized from THF/MeOH to give 4.021 g tert-butyl 4-[6-[[7-cyclopentyl-6-(dimethylcarbamoyl)pyrrolo[2,3-d]pyrimidin-2-yl]amino]-3-pyridyl]piperazine-1-carboxylate of formula I with 99.50 % HPLC purity (85 % overall yield).

### Example 1-4:

A mixture of 0.997 g of tert-butyl 4-(6-aminopyridin-3-yl)piperazine-1-carboxylate of formula III (97 % NMR assay) in 12 mL THF was cooled to about 0 °C under inert atmosphere. 0.842 g of KOtBu in 3.8 mL THF were added and the resulting mixture was stirred at about 0 °C under inert atmosphere. The mixture was combined with 1.00 g of 2-chloro-7-cyclopentyl-N,N-dimethyl-7H-pyrrolo[2,3-d]pyrimidine-6-carboxamide of formula II (97 % NMR assay) in 9 mL THF at about 0 °C, and the resulting reaction mixture was stirred at about 0 °C under inert atmosphere for about 3 h.

The reaction mixture was warmed to RT and 3.15 mL of water are added. After about 1.5 h of stirring at RT, another 18.25 mL of water were added and the mixture was stirred at RT for about 2.5 h. THF was then evaporated and the resulting mixture was stirred at RT for about 1 h. The mixture was filtered and the cake was washed with 10 mL of water. After drying overnight at RT, 1.814 g of crude product were obtained with 95.63 % HPLC purity.

### Example 1-5:

A mixture of 0.997 g of tert-butyl 4-(6-aminopyridin-3-yl)piperazine-1-carboxylate of formula III (97 % NMR assay) in 12 mL THF was cooled to about 0 °C under inert atmosphere. 0.843 g of KOtBu in 6.6 mL THF were added and the resulting mixture was stirred at about 0 °C under inert atmosphere. The mixture was combined with 1.00 g of 2-chloro-7-cyclopentyl-N,N-dimethyl-7H-pyrrolo[2,3-d]pyrimidine-6-carboxamide of formula II (97 % NMR assay) in 9 mL THF at about 0 °C, and the resulting reaction mixture was stirred at about 0 °C under inert atmosphere for about 2.5 h.

The reaction mixture was warmed to RT and 21 mL of water were added and the mixture was stirred at RT for about 5.5 h. THF was then evaporated and the resulting mixture was stirred at RT for about 1 h. The mixture was filtered and the cake was washed with 10 mL of water. After drying overnight at RT, 1.905 g of crude product were obtained with 96.10 % HPLC purity.

### Example 1-6:

A mixture of 2.991 g of tert-butyl 4-(6-aminopyridin-3-yl)piperazine-1-carboxylate of formula III (97 % NMR assay) in 36 mL THF was cooled to about 0 °C under inert atmosphere. 2.53 g of KOtBu in 20 mL THF were added and the resulting mixture was stirred at about 0 °C under inert atmosphere. The mixture was combined with 3.00 g of 2-chloro-7-cyclopentyl-N,N-dimethyl-7H-pyrrolo[2,3-d]pyrimidine-6-carboxamide of formula II (97 % NMR assay) in 27 mL THF at about 0 °C, and the resulting reaction mixture was stirred at about 0 °C under inert atmosphere for about 4 h.

Next, 63 mL of water were added at about 0 °C. After about 3 h of stirring at about 0 °C, the mixture was warmed to RT and stirred at RT for about 3.5 h. THF was then evaporated and the resulting mixture was stirred at RT for about 0.5-1 h. The mixture was filtered and the cake was washed with 30 mL of water. After drying overnight at RT, 5.470 g of crude product were obtained with 96.19 % HPLC purity.

### Example 1-7:

A mixture of 0.997 g of tert-butyl 4-(6-aminopyridin-3-yl)piperazine-1-carboxylate of formula III (97 % NMR assay) in 12 mL THF was cooled to about 0 °C under inert atmosphere. 0.889 g of KOtBu in 6.9 mL THF were added and the resulting mixture was stirred at about 0 °C under inert atmosphere. The mixture was combined with 1.00 g of 2-chloro-7-cyclopentyl-N,N-dimethyl-7H-pyrrolo[2,3-d]pyrimidine-6-carboxamide of formula II (97 % NMR assay) in 9 mL THF at about 0 °C, and the resulting reaction mixture was stirred at about 0 °C under inert atmosphere for about 2.5 h.

Next, 21 mL of water were added at about 0 °C. The mixture was warmed to RT and stirred at RT for about 2 h. THF was then evaporated and the resulting mixture was stirred at RT for about 1 h. The mixture was filtered and the cake was washed with 10 mL of water. After drying overnight at RT, 1.799 g of crude product were obtained with 96.46 % HPLC purity.

### Example 1-8:

A mixture of 9.97 g of tert-butyl 4-(6-aminopyridin-3-yl)piperazine-1-carboxylate of formula III (97 % NMR assay) in 120 mL THF was cooled to about 0 °C under inert atmosphere. 8.89 g of KOtBu in 69 mL THF were added and the resulting mixture was stirred at about 0 °C under inert atmosphere. The mixture was combined with 10.0 g of 2-chloro-7-cyclopentyl-N,N-dimethyl-7H-pyrrolo[2,3-d]pyrimidine-6-carboxamide of formula II (97 % NMR assay) in 90 mL THF at about 0 °C, and the resulting reaction mixture was stirred at about 0 °C under inert atmosphere for about 2.5 h.

Next, 210 mL of water were added at about 0 °C. The mixture was warmed to RT and stirred at RT for about 7.5 h. THF was then evaporated and the resulting mixture was stirred at RT for about 1 h. The mixture was filtered and the cake was washed with 100 mL of water. After drying at 35 °C, 18.275 g of crude product were obtained with 97.80 % HPLC purity.

13.839 g of the crude product was recrystallized from THF/MeOH to give 11.427 g tert-butyl 4-[6-[[7-cyclopentyl-6-(dimethylcarbamoyl)pyrrolo[2,3-d]pyrimidin-2-yl]amino]-3-pyridyl]piperazine-1-carboxylate of formula I with % HPLC purity of 99.53% (83 % overall yield).

### Example 1-9:

A mixture of 0.997 g of tert-butyl 4-(6-aminopyridin-3-yl)piperazine-1-carboxylate of formula III (97 % NMR assay) in 12 mL THF was cooled to about 0 °C under inert atmosphere. 2.67 g of KOtBu in 21 mL THF were added and the resulting mixture was stirred at about 0 °C under inert atmosphere. The mixture was combined with 1.00 g of 2-chloro-7-cyclopentyl-N,N-dimethyl-7H-pyrrolo[2,3-d]pyrimidine-6-carboxamide of formula II (97 % NMR assay) in 9 mL THF at about 0 °C, and the resulting reaction mixture was stirred at about 0 °C under inert atmosphere for about 3 h.

Next, 21 mL of water were added at about 0 °C. The mixture was warmed to RT and stirred at RT for about 8.5 h. THF was then evaporated and the resulting mixture was stirred at RT for about 1 h. The mixture was filtered and the cake was washed with 10 mL of water. After drying at 35 °C, 1.674 g of crude product were obtained with 96.69 % HPLC purity.

Selected experimental conditions regarding step (b) of synthetic examples 1-1 to 1-8 are compiled in the table hereinafter:

| | educt (III) | | base KOtBu | | educt (II) | | time | temp. | purity¹ | yield² |
|---|---|---|---|---|---|---|---|---|---|---|
| | [g] | solvent | [g] | solvent | [g] | solvent | [h] | [°C] | [%] | [%] |
| 1-1 | 1.14 | toluene | 0.767 | - | 1.00 | toluene | ≥3 | 23 | 96.12 | 70.95 |
| 1-2 | 3.42 | THF | 2.53 | THF | 3.00 | THF | 2 | 0 | 96.10 | 85² |
| 1-3 | 6.84 | THF | 5.06 | THF | 6.00 | THF | 2 | 0 | 93.42 | 85² |
| 1-4 | 0.997 | THF | 0.842 | THF | 1.00 | THF | 3 | 0 | 95.63 | |
| 1-5 | 0.997 | THF | 0.843 | THF | 1.00 | THF | 2.5 | 0 | 96.10 | |
| 1-6 | 2.991 | THF | 2.53 | THF | 3.00 | THF | 4 | 0 | 96.19 | |
| 1-7 | 0.997 | THF | 0.889 | THF | 1.00 | THF | 2.5 | 0 | 96.46 | |
| 1-8 | 9.97 | THF | 8.89 | THF | 10.0 | THF | 2.5 | 0 | 97.80 | 83² |
| 1-9 | 0.997 | THF | 2.67 | THF | 1.00 | THF | 3 | 0 | 96.69 | |

| | | | | | | | | | | |
|---|---|---|---|---|---|---|---|---|---|---|
| RT room temperature; ¹ before recrystallization; ² after recrystallization | | | | | | | | | | |

Adding water to the reaction mixture significantly improves the product yield and process performance. When no water is added, e.g. after 3 h at 0 °C and subsequent warming to about 2 h at 40 °C, the yield of the desired product (i.e. the relative amount of product in the reaction mixture according to HPLC results) is very poor, whereas with addition of water the desired product is obtained in excellent yield, as demonstrated by Examples 1-1 to 1-9.

### Example 1-10: no addition of water

A mixture of 0.997 g of tert-butyl 4-(6-aminopyridin-3-yl)piperazine-1-carboxylate of formula III (97 % NMR assay) in 12 mL THF was cooled to about 0 °C under inert atmosphere. 0.889 g of KOtBu in 6.9 mL THF were added and the resulting mixture was stirred at about 0 °C under inert atmosphere. The mixture was combined with 1.00 g of 2-chloro-7-cyclopentyl-N,N-dimethyl-7H-pyrrolo[2,3-d]pyrimidine-6-carboxamide of formula II (97 % NMR assay) in 9 mL THF at about 0 °C, and the resulting reaction mixture was stirred at about 0 °C under inert atmosphere for about 2.5 h.

The reaction mixture was then evaporated. 3.643 g of crude product were obtained with up to 4.22 % HPLC purity.

### Example 1-11: stirring or direct evaporation

A mixture of 1.994 g of tert-butyl 4-(6-aminopyridin-3-yl)piperazine-1-carboxylate of formula III (97 % NMR assay) in 24 mL THF was cooled to about 0 °C under inert atmosphere. 1.78 g of KOtBu in 14 mL THF were added and the resulting mixture was stirred at about 0 °C under inert atmosphere. The mixture was combined with 2.00 g of 2-chloro-7-cyclopentyl-N,N-dimethyl-7H-pyrrolo[2,3-d]pyrimidine-6-carboxamide of formula II (97 % NMR assay) in 18 mL THF at about 0 °C, and the resulting reaction mixture was stirred at about 0 °C under inert atmosphere for about 3 h.

Next, 42 mL of water were added at about 0 °C. The mixture was warmed to RT and proceeded as follows:

(1): 20 mL of the mixture were warmed to 35 °C and stirred at 35 °C for about 1.5 - 2 h. THF was then evaporated and the resulting mixture was stirred at RT for about 1 h. The mixture was filtered and the cake was washed with 5 mL of water. After drying at 35 °C, 0.722 g of crude product were obtained with 97.44 % HPLC purity .

(2): 20 mL of the mixture were warmed to 50 °C and stirred at 50 °C for about 0.5 h. THF was then evaporated and the resulting mixture was stirred at RT for about 1 h. The mixture was filtered and the cake was washed with 5 mL of water. After drying at 35 °C, 0.707 g of crude product were obtained with 97.30 % HPLC purity .

(3): THF was evaporated from about 16 mL of the mixture and the resulting mixture was stirred at RT for about 0.5 - 1 h. The mixture was filtered and the cake was washed with 5 mL of water. After drying at 35 °C, 0.411 g of crude product were obtained with 96.06 % HPLC purity.

### Example 1-12: prolonged heating at 40 °C before addition of water

A mixture of 0.997 g of tert-butyl 4-(6-aminopyridin-3-yl)piperazine-1-carboxylate of formula III (97 % NMR assay) in 12 mL THF was cooled to about 0 °C under inert atmosphere. 0.889 g of KOtBu in 6.9 mL THF were added and the resulting mixture was stirred at about 0 °C under inert atmosphere. The mixture was combined with 1.00 g of 2-chloro-7-cyclopentyl-N,N-dimethyl-7H-pyrrolo[2,3-d]pyrimidine-6-carboxamide of formula II (97 % NMR assay) in 9 mL THF at about 0 °C, and the resulting reaction mixture was stirred at about 0 °C under inert atmosphere for about 2 h. The reaction mixture was warmed to 40 °C and stirred at 40 °C for about 19 - 20 h.

Next, 21 mL of water were added at about 35 °C and the mixture was stirred at 35 °C for about 4 h. THF was then evaporated and the resulting mixture was stirred at RT for about 1 h. The mixture was filtered and the cake was washed with 20 mL of water. After drying at 35 °C, 1.476 g of crude product were obtained with 95.49 % HPLC purity.

### Example 2 - Synthesis of Ribociclib

1.000 g of tert-butyl 4-[6-[[7-cyclopentyl-6-(dimethylcarbamoyl)pyrrolo[2,3-d]pyrimidin-2-yl]amino]-3-pyridyl]piperazine-1-carboxylate of formula I was combined with 2.34 mL of 5 M HCl (aq.). The reaction mixture was stirred at about 25 °C for about 2.5 h. Then the pH was adjusted with 30 % NaOH (aq.) to about 13.8. The mixture was stirred at RT for about 0.5 h, filtered and the cake was washed with 20 mL of water. After drying at 50 °C, 0.651 g of crude product were obtained with 99.39 % HPLC purity.

### Example 3 - Synthesis of salts of Ribociclib (acid addition salt):

General preparation procedure for salts of Ribociclib:
1) dissolving or suspending Ribociclib (non-salt form, free base) in a solvent A and optionally a solvent B, e.g. in an organic solvent, in a mixture of two organic solvents, or in a mixture of an organic solvent with water;
2) adding selected acid in solid form or dissolved in a solvent C and optionally a solvent D, e.g. in an organic solvent, in a mixture of two organic solvents, or in a mixture of an organic solvent with water;
3) optionally, heating the thus obtained reaction mixture from room temperature to reflux temperature thereby forming the acid addition salt in suspension and/or solution;
4) optionally, adding solvent C and/or solvent D to said acid addition salt in suspension and/or solution;
5) optionally, cooling the mixture;
6) optionally, evaporating at least a portion of the first and/or second solvent system; and
7) optionally, isolating the acid addition salt.

### Analytical methods

¹H NMR and ¹³C NMR spectra were recorded on Bruker AVANCE NEO 600 MHz NMR spectrometer (Solvent: DMSO-d6, Temperature: 25°C).

IR spectra were recorded on Perkin Elmer Frontier FT-IR spectrometer using KBr pellets (Ph. Eur. 2.2.24.).

Unless expressly stated otherwise, process control and product purity was assessed by high pressure liquid chromatography (HPLC):

| | |
|---|---|
| Column: | YMC Triart C18; (150 x 4.6) mm; 3 µm particles |
| Mobile phase A: | 0,01 M (NH₄)H₂PO₄, pH = 3.0 |
| Mobile phase B: | methanol |

Gradient:

| Time (min) | Mobile phase A (%) | Mobile phase B (%) |
|---|---|---|
| 0 | 90 | 10 |
| 5 | 50 | 50 |
| 15 | 10 | 90 |
| 20 | 10 | 90 |
| 21 | 90 | 10 |
| 30 | 90 | 10 |

| | |
|---|---|
| Column temperature: | 45 °C |
| Flow: | 0.7 ml/min |
| Detection: | UV, wavelength 230 nm |
| Injection volume: | 5 µl |

In Examples 1-2 to 1-12 and Example 2 product purity was assessed by high pressure liquid chromatography (HPLC):

| | |
|---|---|
| Column: | YMC Triart C18; (150 x 4.6) mm; 3 µm particles |
| Mobile phase A: | 0.01 M (NH₄)₂HPO₄, pH = 7.0 |
| Mobile phase B: | 90 % acetonitrile (V/V) |

Gradient:

| Time (min) | Mobile phase A (%) | Mobile phase B (%) |
|---|---|---|
| 0 | 85 | 15 |
| 17 | 30 | 70 |
| 25 | 20 | 80 |
| 35 | 20 | 80 |
| 36 | 85 | 15 |
| 40 | 85 | 15 |

| | |
|---|---|
| Column temperature: | 40°C |
| Flow: | 1.0 ml/min |
| Detection: | UV, wavelength 230 nm |
| Injection volume: | 5 µl |

Identification of cations and anions was determined by mass spectrometry (LC-MS and GC-MS):
LC-MS method:

| | |
|---|---|
| Column: | YMC Triart C18; (150 x 4.6) mm; 3 µm particles |
| Mobile phase A: | 0,005 M (NH₄)HCOO, pH = 3.0 |
| Mobile phase B: | methanol |

Gradient:

| Time (min) | Mobile phase A (%) | Mobile phase B (%) |
|---|---|---|
| 0 | 90 | 10 |
| 5 | 50 | 50 |
| 15 | 10 | 90 |
| 20 | 10 | 90 |
| 21 | 90 | 10 |
| 30 | 90 | 10 |

| | |
|---|---|
| Column temperature: | 45 °C |
| Flow: | 0.7 ml/min |
| Detection: | UV, wavelength 230 nm |
| Injection volume: | 5 µl |

Ionization: ESI
GC-MS method:

| | |
|---|---|
| Column | Rtx1071 |
| Length | 30 m |
| ID | 0.25 mm |
| Carrier gas | Helium |
| Carrier gas flow rate | 1.2 ml/min (constant flow) |
| Split ratio | 20:1 |
| Make-up gas N2 flow rate | 25 ml/min |
| Air flow rate | 400 ml/min |
| Hydrogen flow rate | 40 ml/min |
| Temperature program | 60°C (5 min) → 10°C/min → 260°C (5 min) |
| Injector temperature | 200°C |
| Detector temperature | 200°C |
| Injection volume | 1.0 µL |

Ionization EI

### Example 3-1 - salt of Ribociclib with malonic acid

Ribociclib (0.50 g) was suspended in a mixture dichloromethane (4 mL) and methanol (1 mL). Malonic acid (0.13 g) was added at 30 °C. A clear solution was obtained. The solution was cooled and methanol (3 mL) was added thereby inducing precipitation. The precipitated material was isolated and dried in a vacuum drier at 22°C. Yield 0.25 g. HPLC (purity): 99.87 %. MS(ESI) m/z 435.26 (M+H)+ Ribociclib base (C₂₃H₃₁N₈O⁺). MS(ESI) m/z 103.00 (M-H)- Malonic acid (C₃H₃O₄⁻)

¹H NMR (DMSO-d₆) δ: 9.46 (s, 1H), 8.78 (s, 1H), 8.19 (d, 1H), 8.05 (d, 1H), 7.48 (m, 1H), 6.60 (s, 1H), 4.74 (m, 1H), 3.22-3.32 (m, 4H), 3.13-3.20 (m, 4H), 3.01-3.09 (m, 6H), 2.72 (s, 2H), 2.37-2.48 (m, 2H), 1.91-2.04 (m, 4H), 1.58-1.72 (m, 2H).

¹³C NMR (DMSO-d₆) δ: 171.6, 162.9, 154.7, 152.1, 151.2, 146.8, 141.6, 136.1, 131.9, 125.9, 112.5, 111.9, 100.6, 56.9, 47.1, 43.4, 38.8, 34.6, 29.7, 24.2.

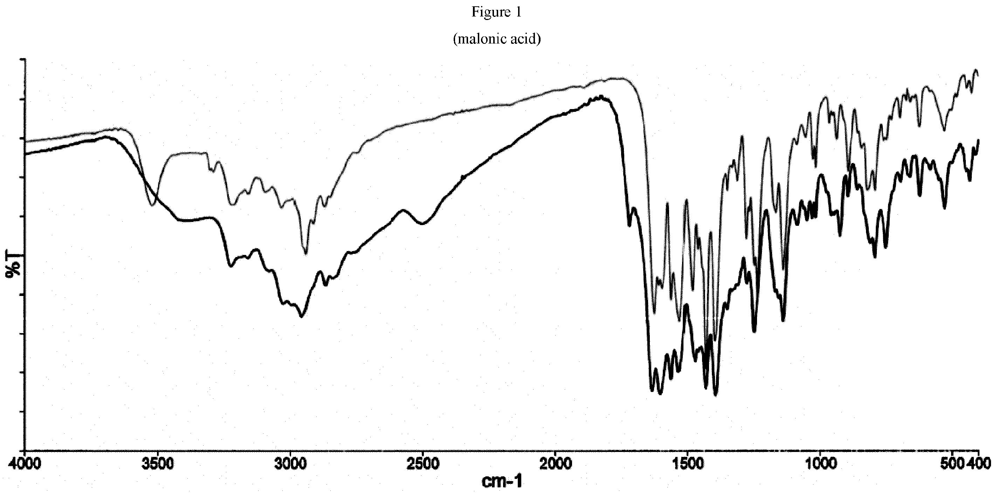

IR (KBr pellet; Figure 1 shows an overlay plot of IR spectra, upper spectrum represents Ribociclib base, lower spectrum represents newly prepared Ribociclib salt with malonic acid).

### Example 3-2 - salt of Ribociclib with cinnamic acid

Ribociclib (0.50 g) was suspended in 50 % aqueous isopropanol (2.5 mL). Cinnamic acid (0.18 g) was added at 55 °C. A clear solution was obtained followed by spontaneous precipitation at 55 °C. The solution was cooled and the precipitated material was isolated and dried in vacuum drier at 22 °C. Yield 0.86 g. HPLC (purity): 100.00%. MS(ESI) m/z 435.26 (M+H)+ Ribociclib base (C₂₃H₃₁N₈O⁺). MS(ESI) m/z 147.04 (M-H)- Cinnamic acid (C₉H₇O₂⁻).

¹H NMR (DMSO-d₆) δ: 9.34 (s, 1H), 8.76 (s, 1H), 8.15 (d, 1H), 8.00 (d, 1H), 7.65 (m, 2H), 7.51 (d, 1H), 7.36-7.45 (m, 4H), 6.59 (s, 1H), 6.52 (d, 1H), 4.73 (m, 1H), 2.99-3.14 (m, 10H), 2.87-2.95 (m, 4H), 2.36-2.47 (m, 2H), 1.91-2.04 (m, 4H), 1.58-1.70 (m, 2H).

¹³C NMR (DMSO-d₆) δ: 168.2, 162.9, 154.8, 152.1, 151.2, 146.1, 142.7, 142.5, 135.7, 134.6, 131.8, 129.9, 128.9, 128.0, 125.3, 121.0, 112.6, 111.8, 100.6, 56.9, 49.3, 44.9, 38.8, 34.6, 29.7, 24.2.

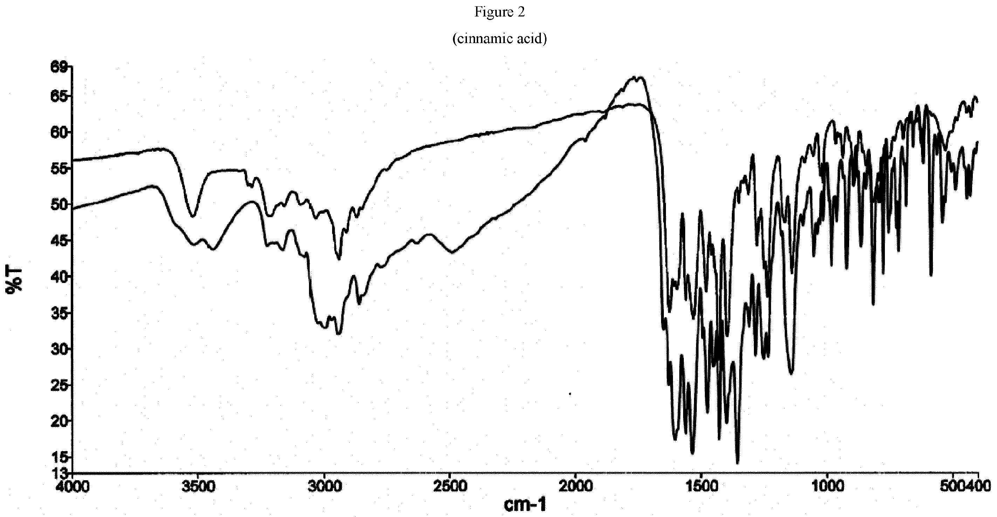

IR (KBr pellet; Figure 2 shows an overlay plot of IR spectra, upper spectrum represents Ribociclib base, lower spectrum represents newly prepared Ribociclib salt with cinnamic acid).

### Example 3-3 - salt of Ribociclib with phthalic acid

Ribociclib (0.50 g) was suspended in isopropanol (1.25 mL). Phthalic acid (0.18 g) dissolved in 1.25 mL of water was added at 55 °C. A clear solution was obtained followed by spontaneous precipitation at 55 °C. The solution was cooled and the precipitated material was isolated and dried in vacuum drier at 22 °C. Yield 0.32 g. HPLC (purity): 99.19 %. MS(ESI) m/z 435.26 (M+H)+ Ribociclib base (C₂₃H₃₁N₈O⁺). MS(ESI) m/z 165.01 (M-H)- Phthalic acid (C₈H₅O₄⁻).

¹H NMR (DMSO-d₆) δ: 9.44 (s, 1H), 8.77 (s, 1H), 8.20 (d, 1H), 8.14-8.17 (m, 2H), 8.06 (d, 1H), 7.47-7.53 (m, 3H), 6.60 (s, 1H), 4.74 (m, 1H), 3.30-3.33 (m, 4H), 3.24-3.27 (m, 4H), 2.95-3.12 (m, 6H), 2.35-2.47 (m, 2H), 1.91-2.04 (m, 4H), 1.58-1.71 (m, 2H).

¹³C NMR (DMSO-d₆) δ: 168.0, 162.9, 154.7, 152.1, 151.1, 147.0, 141.2, 136.3, 135.0, 132.4, 132.0, 130.3, 126.1, 112.6, 111.9, 100.6, 56.9, 46.4, 42.9, 38.7, 34.6, 29.7, 24.2.

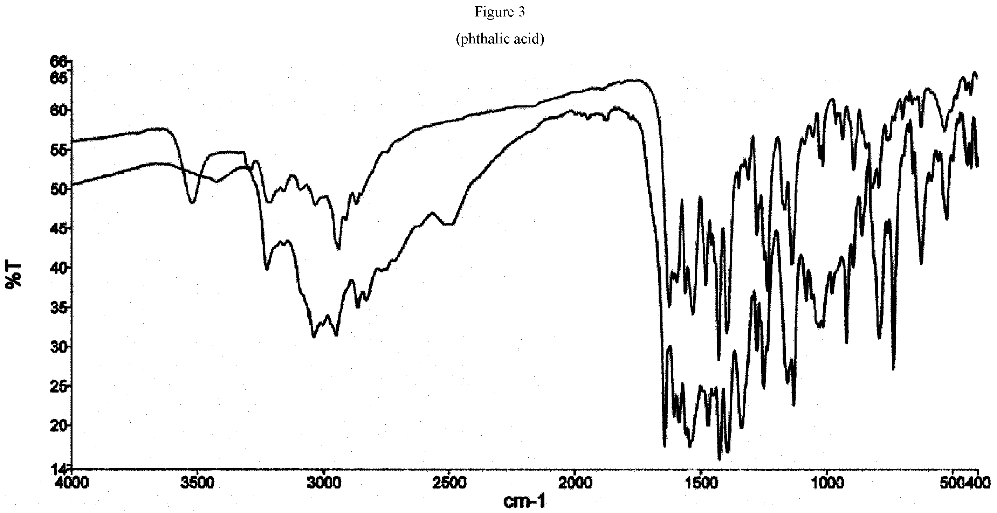

IR (KBr pellet; Figure 3 shows an overlay plot of IR spectra, upper spectrum represents Ribociclib base, lower spectrum represents newly prepared Ribociclib salt with phthalic acid).

### Example 3-4 - salt of Ribociclib with glutamic acid

Ribociclib (0.50 g) was suspended in a mixture dichloromethane (4 mL) and methanol (1 mL). Glutamic acid (0.18 g) was added at 30 °C. A clear solution was obtained. The solution was cooled and methanol (2 mL) was added thereby inducing precipitation. Precipitated material was isolated and dried in vacuum drier at 22°C. Yield 0.21 g. HPLC (purity): 99,88 %. MS(ESI) m/z 435.26 (M+H)⁺ Ribociclib base (C₂₃H₃₁N₈O⁺). MS(ESI) m/z 146.04 (M-H)⁻ Glutamic acid (C₅H₈NO₄⁻).

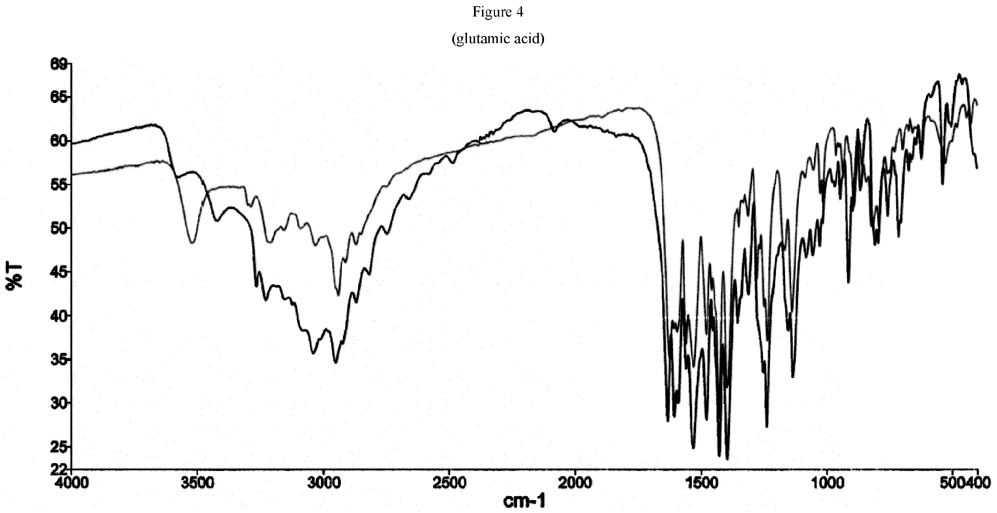

IR (KBr pellet; Figure 4 shows an overlay plot of IR spectra, upper spectrum represents Ribociclib base, lower spectrum represents newly prepared Ribociclib salt with glutamic acid).

### Example 3-5 - salt of Ribociclib with glutaric acid

Ribociclib (0.50 g) was suspended in a mixture dichloromethane (4 mL) and methanol (1 mL). Glutaric acid (0.16 g) was added at 30 °C. A clear solution was obtained. The solution was cooled and methanol (2 mL) was added thereby inducing precipitation. The precipitated material was isolated and dried in vacuum drier at 22°C. Yield 0.54 g. HPLC (purity): 100.00 %. MS(ESI) m/z 435.26 (M+H)⁺ Ribociclib base (C₂₃H₃₁N₈O⁺). MS(ESI) m/z 131.03 (M-H)⁻ Glutaric acid (C₅H₇O₄⁻).

¹H NMR (DMSO-d₆) δ: 9.33 (s, 1H), 8.76 (s, 1H), 8.15 (d, 1H), 7.99 (d, 1H), 7.42 (m, 1H), 6.59 (s, 1H), 4.73 (m, 1H), 2.99-3.11 (m, 10H), 2.88-2.93 (m, 4H), 2.37-2.48 (m, 2H), 2.22 (m, 4H), 1.92-2.01 (m, 4H), 1.69 (m, 2H), 1.60-1.67 (m, 2H).

¹³C NMR (DMSO-d₆) δ: 174.3, 162.9, 154.8, 152.1, 151.2, 146.1, 142.7, 135.6, 131.8, 125.3, 112.6, 111.8, 100.6, 56.9, 49.3, 45.0, 38.8, 34.6, 33.2, 29.7, 24.2, 20.2.

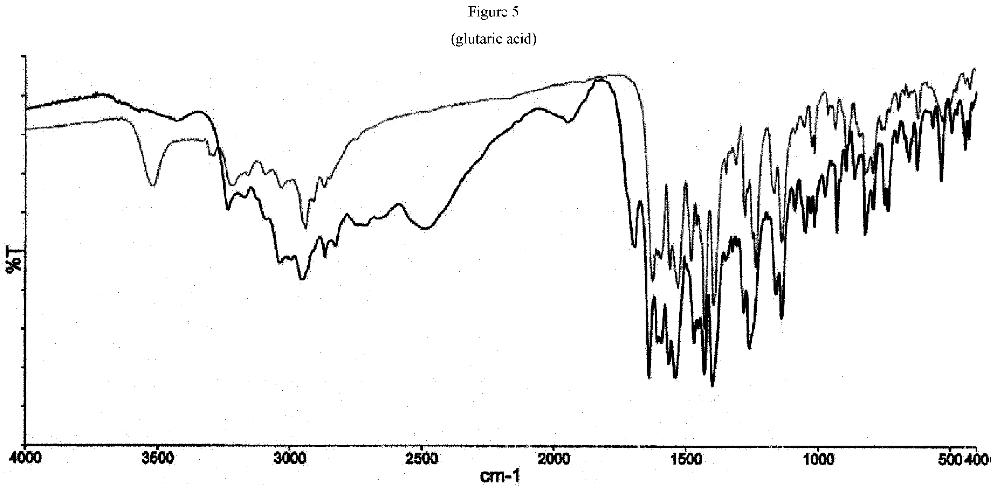

IR (KBr pellet; Figure 5 shows an overlay plot of IR spectra, upper spectrum represents Ribociclib base, lower spectrum represents newly prepared Ribociclib salt with glutaric acid).

### Example 3-6 - salt of Ribociclib with propionic acid

Ribociclib (0.50 g) was suspended in a mixture dichloromethane (4 mL) and methanol (1 mL). Propionic acid (90 µL) was added at 30 °C. A clear solution was obtained. The solution was cooled and methanol (4 mL) was added. Dichloromethane was exchanged with ethyl acetate (4 mL). The mixture ethyl acetate/ methanol was concentrated thereby inducing precipitation. The precipitated material was isolated and dried in vacuum drier at 22°C. Yield 0.52 g. HPLC (purity): 99.92 %. MS(ESI) m/z 435.26 (M+H)⁺ Ribociclib base (C₂₃H₃₁N₈O⁺). GC-MS m/z 74.04 Propionic acid (C₃H₆O₂).

¹H NMR (DMSO-d₆) δ: 9.36 (s, 1H), 8.77 (s, 1H), 8.14 (d, 1H), 7.99 (d, 1H), 7.41 (m, 1H), 6.59 (s, 1H), 4.73 (m, 1H), 2.97-3.12 (m, 10H), 2.82-2.89 (m, 4H), 2.36-2.47 (m, 2H), 2.19 (m, 2H), 1.91-2.05 (m, 4H), 1.56-1.72 (m, 2H), 0.98 (m, 3H).

¹³C NMR (DMSO-d₆) δ: 175.3, 162.9, 154.8, 152.1, 151.2, 146.0, 142.9, 135.5, 131.8, 125.2, 112.6, 111.8, 100.6, 56.9, 49.8, 45.3, 38.8, 34.6, 29.7, 27.1, 24.2, 9.2.

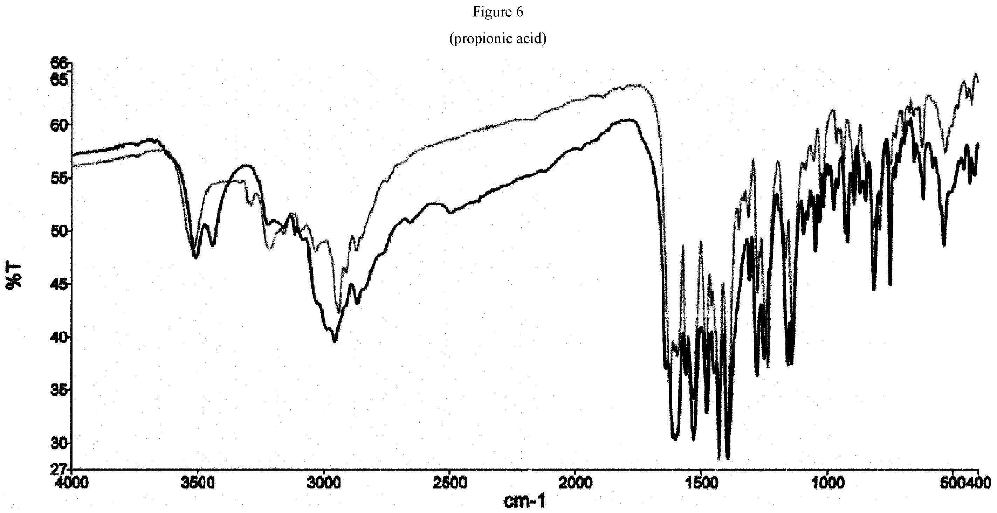

IR (KBr pellet; Figure 6 shows an overlay plot of IR spectra, upper spectrum represents Ribociclib base, lower spectrum represents newly prepared Ribociclib salt with propionic acid).

### Example 3-7 - salt of Ribociclib with formic acid

Ribociclib (0.50 g) was suspended in a mixture dichloromethane (4 mL) and methanol (1 mL). Formic acid (46 µL) was added at 30 °C. A clear solution was obtained. The solution was cooled and methanol (4 mL) was added. Dichloromethane was exchanged with ethyl acetate (4 mL) thereby inducing precipitation. The precipitated material was isolated and dried in vacuum drier at 22°C. Yield 0.57 g. HPLC (purity): 99.83 %. MS(ESI) m/z 435.26 (M+H)⁺ Ribociclib base (C₂₃H₃₁N₈O⁺). GC-MS m/z 46.01 Formic acid (CH₂O₂).

¹H NMR (DMSO-d₆) δ: 9.37 (s, 1H), 8.77 (s, 1H), 8.29 (s, 1H), 8.16 (d, 1H), 8.01 (d, 1H), 7.44 (m, 1H), 6.60 (s, 1H), 4.73 (m, 1H), 3.10-3.20 (m, 5H), 3.02-3.08 (m, 6H), 2.96-3.02 (m, 4H), 2.36-2.47 (m, 2H), 2.04-1.90 (m, 4H), 1.57-1.71 (m, 2H).

¹³C NMR (DMSO-d₆) δ: 164.5, 162.9, 154.7, 152.1, 151.2, 146.3, 142.3, 135.8, 131.9, 125.5, 112.6, 111.8, 100.6, 56.9, 48.5, 44.2, 38.8, 34.6, 29.7, 24.2.

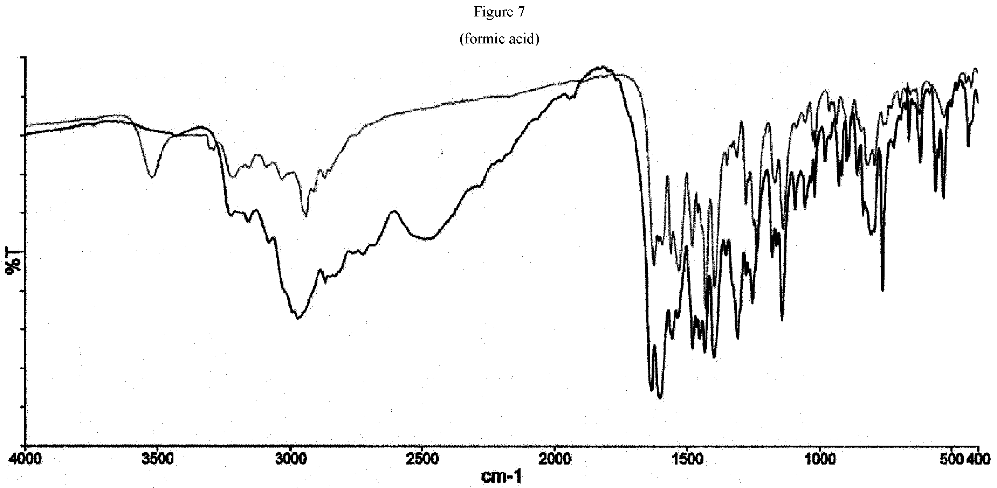

IR (KBr pellet; Figure 7 shows an overlay plot of IR spectra, upper spectrum represents Ribociclib base, lower spectrum represents newly prepared Ribociclib salt with formic acid).

## Claims

1. A process for preparing a 4-[6-[[7-cyclopentyl-6-(dimethylcarbamoyl)pyrrolo[2,3-d]pyrimidin-2-yl]amino]-3-pyridyl]piperazine-1-carboxylate of formula (I) 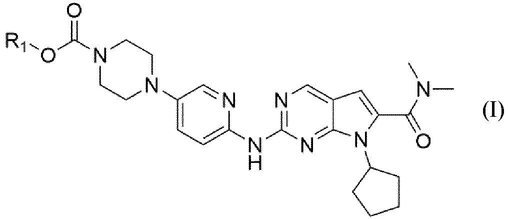 wherein R1 represents -C₁₋₁₂-alkyl, -C₁₋₁₂-aryl, or -C₁₋₁₂-alkyl-C₁₋₁₆-aryl;
the process comprising the steps of:
(a) providing a composition comprising
(i) a 4-(6-aminopyridin-3-yl)piperazine-1-carboxylate of formula (III) 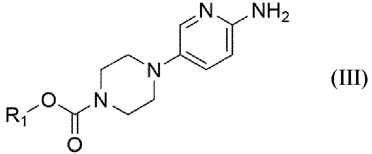 wherein R1 represents -C₁₋₁₂-alkyl, -C₁₋₁₂-aryl, or -C₁₋₁₂-alkyl-C₁₋₁₆-aryl;
(ii) a solvent which is selected from the group consisting of n-pentane, n-hexane, cyclohexane, methyl cyclohexane, toluene, benzene, xylene, dichloromethane, tetrahydrofuran, and 1,4-dioxane; and
(iii) a base;
- wherein the conjugate acid of the base has a pKa value of at most 25; and
- wherein the base has general formula (IV) 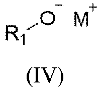 wherein M is selected from Li, Na, and K; and R₁ in general formula (IV) is selected from the group consisting of methyl, ethyl, -tert-butyl, -benzyl, -methylene-fluorenyl, and -allyl;
(b) adding to the composition provided in step (a) a 7-cyclopentyl-N,N-dimethyl-7H-pyrrolo[2,3-d]pyrimidine-6-carboxamide of formula (II) 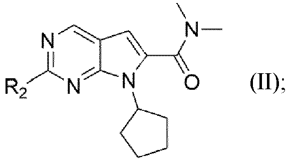 wherein R2 represents a leaving group;
(c) allowing the composition obtained in step (b) to react thereby obtaining a composition comprising the 4-[6-[[7-cyclopentyl-6-(dimethylcarbamoyl)pyrrolo[2,3-d]pyrimidin-2-yl]amino]-3-pyridyl]piperazine-1-carboxylate of formula (I);
(d) optionally, adding an additional amount of base; preferably of the same base that was also contained in the composition provided in step (a);
(e) optionally, adding water to the composition obtained in step (c) or (d); and
(f) optionally, recovering the 4-[6-[[7-cyclopentyl-6-(dimethylcarbamoyl)pyrrolo[2,3-d]pyrim-idin-2-yl]amino]-3-pyridyl]piperazine-1-carboxylate of formula (I) from the composition obtained in step (c), (d) or (e).

2. The process according to claim 1, wherein the stoichiometric ratio (mole : mole) of the 4-(6-aminopyridin-3-yl)piperazine-1-carboxylate of formula (III) to the a 7-cyclopentyl-N,N-dimethyl-7H-pyrrolo[2,3-d]pyrimidine-6-carboxamide of formula (II) is at least 0.9, preferably at least 1.0, more preferably at least 1.05, still more preferably at least 1.1, even more preferably at least 1.2.

3. The process according to claim 1 or 2, wherein the stoichiometric ratio (mole : mole) of the 4-(6-aminopyridin-3-yl)piperazine-1-carboxylate of formula (III) to the a 7-cyclopentyl-N,N-dimethyl-7H-pyrrolo[2,3-d]pyrimidine-6-carboxamide of formula (II) is at most 2.0, preferably at most 1.9, more preferably at most 1.8, still more preferably at most 1.7, yet more preferably at most 1.6, even more preferably at most 1.5, most preferably at most 1.4.

4. The process according to any of the preceding claims, wherein the stoichiometric ratio (mole : mole) of the 4-(6-aminopyridin-3-yl)piperazine-1-carboxylate of formula (III) to the 7-cyclopentyl-N,N-dimethyl-7H-pyrrolo[2,3-d]pyrimidine-6-carboxamide of formula (II) is within the range of 1.20±0.20 : 1.00, more preferably 1.20±0.15 : 1.00, still more preferably 1.20±0.10 : 1.00, and even more preferably 1.20±0.05 : 1.00.

5. The process according to any of the preceding claims, wherein the stoichiometric ratio (mole : mole) of the 4-(6-aminopyridin-3-yl)piperazine-1-carboxylate of formula (III) to the 7-cyclopentyl-N,N-dimethyl-7H-pyrrolo[2,3-d]pyrimidine-6-carboxamide of formula (II) is within the range of 1.05±0.05 : 1.00, more preferably 1.05±0.04 : 1.00, still more preferably 1.05±0.03 : 1.00, and even more preferably 1.05±0.02 : 1.00.

6. The process according to any of the preceding claims, wherein the reaction of the 4-(6-aminopy-ridin-3-yl)piperazine-1-carboxylate of formula (III) and the 7-cyclopentyl-N,N-dimethyl-7H-pyrrolo[2,3-d]pyrimidine-6-carboxamide of formula (II) in step (c) or (d) proceeds via an aromatic nucleophilic substitution (S_{N}Ar) mechanism.

7. The process according to any of the preceding claims, wherein the stoichiometric ratio (mole : mole) of the base relative to the 7-cyclopentyl-N,N-dimethyl-7H-pyrrolo[2,3-d]pyrimidine-6-carboxamide of formula (II) is within the range of 2.3±2.1 : 1.0, preferably 2.3±1.8 : 1.0, more preferably 2.3±1.5 : 1.0, still more preferably 2.3±1.2 : 1.0, yet more preferably 2.3±0.9 : 1.0, even more preferably 2.3±0.6 : 1.0, most preferably 2.3±0.3 : 1.0, and in particular 2.3±0.2 : 1.0.

8. The process according to any of the preceding claims, wherein
- the base is potassium tert-butanolate; and/or
- R1 in general formula (III) is selected from the group consisting of -methyl, -ethyl, -tert-butyl, -benzyl, -methylene-fluorenyl, and -allyl; preferably R1 in general formula (III) is -tert-butyl; and/or
- R2 in general formula (II) is selected from the group consisting of -Cl, -Br, -I, -O-S(=O)₂-CH₃, -O-S(=O)₂-CF₃, and -O-S(=O)₂-(C₆H₄)-CH₃; preferably R2 in general formula (II) is -Cl.

9. The process according to any of the preceding claims, wherein the conjugate acid of the base has a pKa value of at most 20, preferably at most 18.

10. The process according to any of the preceding claims, wherein the solvent additionally contains water that is added for work-up, i.e. after the reaction has taken place.

11. The process according to any of the preceding claims, wherein the solvent is a non-polar aprotic solvent selected from n-pentane, n-hexane, cyclohexane, methyl cyclohexane, toluene, benzene, xylene, tetrahydrofuran, and 1,4-dioxane; preferably toluene or tetrahydrofuran; more preferably tetrahydrofuran; preferably wherein the solvent additionally contains water.

12. The process according to any of the preceding claims, which does not involve
- a copper-catalyzed amination reaction;
- a palladium catalyzed amination reaction; and
- an amination reaction involving an organosilicon base.

13. The process according to any of the preceding claims, wherein the composition obtained in step (b) does not comprise
- copper (I) iodide, preferably no copper salt;
- palladium (II) acetate, [(2-di-cyclohexylphosphino-3,6-dimethoxy-2',4',6'-triisopropyl-1,1'-biphenyl)-2-(2'-amino-1,1'-biphenyl)]palladium (II) methanesulfonate, and tris(dibenzylideneacetone)dipalladium(0); preferably no palladium compound;
- 2,2'-bis(diphenylphosphino)-1,1'-binaphthyl) (BINAP), preferably no organophosphorus ligand;
- cesium carbonate, preferably no cesium base; and/or
- lithium hexamethyldisilazide, sodium hexamethyldisilazide, and potassium hexamethyldisilazide; preferably no hexamethyldisilazide base, more preferably no organosilicon base.

14. A process for preparing Ribociclib 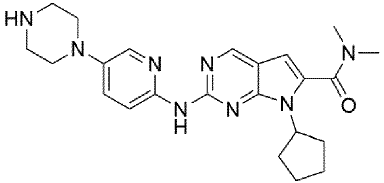 or an acid addition salt thereof, the process comprising a process according to any of the preceding claims and the additional step of:
(g) deblocking the 1N-amino group of the piperazine moiety of the 4-[6-[[7-cyclopentyl-6-(dimethylcarbamoyl)pyrrolo[2,3-d]pyrimidin-2-yl]amino]-3-pyridyl]piperazine-1-carboxylate of formula (I) 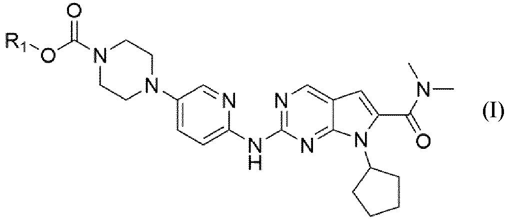 thereby obtaining Ribociclib or an acid addition salt of Ribociclib.

15. A process for preparing a physiologically acceptable salt of Ribociclib comprising a process according to claim 14 and the additional steps of:
(h) providing a composition comprising Ribociclib, an acid, a solvent A and optionally a solvent B; wherein solvent A is preferably selected from alcohols, ketones, esters, and nitriles; and/or solvent B is preferably selected from alkanes, ethers, esters, alcohols and water;
(i) allowing the Ribociclib and the acid to form a physiologically acceptable salt of Ribociclib thereby providing a product composition; and
(j) optionally, recovering at least the majority of the physiologically acceptable salt of Ribociclib from the product composition.

## Patentansprüche

1. Ein Verfahren zur Herstellung eines 4-[6-[[7-Cyclopentyl-6-(dimethylcarbamoyl)pyrrolo[2,3-d]pyrimidin-2-yl]amino]-3-pyridyl]piperazin-1-carboxylats der Formel (I) 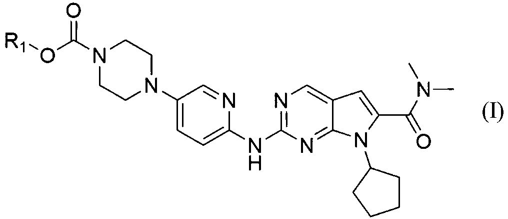 worin R1 -C₁₋₁₂-Alkyl, -C₁₋₁₂-Aryl oder -C₁₋₁₂-Alkyl-C₁₋₁₆-Aryl bedeutet;
wobei das Verfahren die folgenden Schritte umfaßt:
(a) Bereitstellen einer Zusammensetzung, umfassend
(i) ein 4-(6-Aminopyridin-3-yl)piperazin-1-carboxylat der Formel (III) 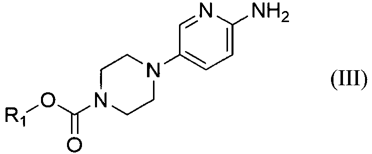 worin R1 -C₁₋₁₂-Alkyl, -C₁₋₁₂-Aryl oder -C₁₋₁₂-Alkyl-C₁₋₁₆-Aryl bedeutet;
(ii) ein Lösungsmittel, das ausgewählt ist aus der Gruppe bestehend aus n-Pentan, n-Hexan, Cyclohexan, Methylcyclohexan, Toluol, Benzol, Xylol, Dichlormethan, Tetrahydrofuran und 1,4-Dioxan; und
(iii) eine Base;
- wobei die konjugierte Säure der Base einen pKa-Wert von höchstens 25 aufweist; und
- wobei die Base die allgemeine Formel (IV) hat 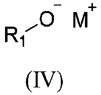 worin M ausgewählt ist aus Li, Na und K; und R₁ in der allgemeinen Formel (IV) ausgewählt ist aus der Gruppe bestehend aus Methyl, Ethyl, -tert-Butyl, -benzyl, -Methylen-Fluorenyl und -Allyl;
(b) Zugabe zu der in Schritt (a) bereitgestellten Zusammensetzung eines 7-Cyclopentyl-N,N-dimethyl-7H-pyrrolo[2,3-d]pyrimidin-6-carboxamids der Formel (II) 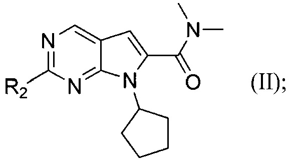 worin R2 eine Abgangsgruppe darstellt;
(c) Reagierenlassen der in Schritt (b) erhaltenen Zusammensetzung, wodurch eine Zusammensetzung erhalten wird, die das 4-[6-[[7-Cyclopentyl-6-(dimethylcarbamoyl)pyrrolo[2,3-d]pyrimidin-2-yl]amino]-3-pyridyl]piperazin-1-carboxylat der Formel (I) umfasst;
(d) gegebenenfalls Zugabe einer zusätzlichen Menge an Base, vorzugsweise der gleichen Base, die auch in der in Schritt (a) bereitgestellten Zusammensetzung enthalten war;
(e) gegebenenfalls Zugabe von Wasser zu der in Schritt (c) oder (d) erhaltenen Zusammensetzung; und
(f) gegebenenfalls die Gewinnung des 4-[6-[[7-Cyclopentyl-6-(dimethylcarbamoyl)pyr-rolo[2,3-d]pyrimidin-2-yl]amino]-3-pyridyl]piperazin-1-carboxylats der Formel (I) aus der in Schritt (c), (d) oder (e) erhaltenen Zusammensetzung.

2. Das Verfahren nach Anspruch 1, wobei das stöchiometrische Verhältnis (Mol : Mol) des 4-(6-Aminopyridin-3-yl)piperazin-1-carboxylats der Formel (III) zu dem 7-Cyclopentyl-N,N-dimethyl-7H-pyrrolo[2,3-d]pyrimidin-6-carboxamid der Formel (II) mindestens 0,9 ist, vorzugsweise mindestens 1,0, stärker bevorzugt mindestens 1,05, noch stärker bevorzugt mindestens 1,1, noch stärker bevorzugt mindestens 1,2.

3. Das Verfahren nach Anspruch 1 oder 2, wobei das stöchiometrische Verhältnis (Mol : Mol) des 4-(6-Aminopyridin-3-yl)piperazin-1-carboxylats der Formel (III) zu dem 7-Cyclopentyl-N,N-dimethyl-7H-pyrrolo[2,3-d]pyrimidin-6-carboxamid der Formel (II) höchstens 2,0 ist, vorzugsweise höchstens 1,9, noch bevorzugter höchstens 1,8, noch bevorzugter höchstens 1,7, noch bevorzugter höchstens 1,6, noch bevorzugter höchstens 1,5, am meisten bevorzugt höchstens 1,4.

4. Das Verfahren nach einem der vorhergehenden Ansprüche, wobei das stöchiometrische Verhältnis (Mol : Mol) des 4-(6-Aminopyridin-3-yl)piperazin-1-carboxylats der Formel (III) zu dem 7-Cyclopentyl-N,N-dimethyl-7H-pyrrolo[2,3-d]pyrimidin-6-carboxamid der Formel (II) im Bereich von 1,20±0,20 : 1,00 liegt, noch bevorzugter 1,20±0,15 : 1,00, noch bevorzugter 1,20±0,10 : 1,00 und noch bevorzugter 1,20±0,05 : 1,00.

5. Das Verfahren nach einem der vorhergehenden Ansprüche, wobei das stöchiometrische Verhältnis (Mol : Mol) des 4-(6-Aminopyridin-3-yl)piperazin-1-carboxylats der Formel (III) zu dem 7-Cyclopentyl-N,N-dimethyl-7H-pyrrolo[2,3-d]pyrimidin-6-carboxamid der Formel (II) im Bereich von 1,05±0,05 : 1,00 liegt, noch bevorzugter 1,05±0,04 : 1,00, noch bevorzugter 1,05±0,03 : 1,00 und noch bevorzugter 1,05±0,02 : 1,00.

6. Das Verfahren nach einem der vorhergehenden Ansprüche, wobei die Reaktion des 4-(6-Aminopyridin-3-yl)piperazin-1-carboxylats der Formel (III) und des 7-Cyclopentyl-N,N-dimethyl-7H-pyrrolo[2,3-d]pyrimidin-6-carboxamids der Formel (II) in Schritt (c) oder (d) über einen aromatischen nukleophilen Substitutionsmechanismus (S_{N}Ar) verläuft.

7. Das Verfahren nach einem der vorhergehenden Ansprüche, wobei das stöchiometrische Verhältnis (Mol : Mol) der Base zum 7-Cyclopentyl-N,N-dimethyl-7H-pyrrolo[2,3-d]pyrimidin-6-car-boxamid der Formel (II) im Bereich von 2,3±2,1 : 1,0 liegt, vorzugsweise 2,3+1,8 : 1,0, noch bevorzugter 2,3±1,5 : 1,0, noch bevorzugter 2,3±1,2 : 1,0, noch bevorzugter 2,3±0,9 : 1,0, noch bevorzugter 2,3±0,6 : 1,0, am meisten bevorzugt 2,3±0,3 : 1,0, und insbesondere 2,3±0,2 : 1,0.

8. Das Verfahren nach einem der vorhergehenden Ansprüche, **dadurch gekennzeichnet, dass**
- die Base Kalium-tert.-butanolat ist; und/oder
- R1 in der allgemeinen Formel (III) ausgewählt ist aus der Gruppe bestehend aus -methyl, - ethyl, -tert-butyl, -benzyl, -methylen-fluorenyl und -allyl; vorzugsweise ist R1 in der allgemeinen Formel (III) -tert-butyl; und/oder
- R2 in der allgemeinen Formel (II) ausgewählt ist aus der Gruppe bestehend aus -Cl, -Br, -I, - O-S(=O)₂-CH₃, -O-S(=O)₂-CF₃ und -O-S(=O)₂ -(C₆ H₄) -CH₃; vorzugsweise ist R2 in der allgemeinen Formel (II) -Cl.

9. Das Verfahren nach einem der vorhergehenden Ansprüche, **dadurch gekennzeichnet, dass** die konjugierte Säure der Base einen pKa-Wert von höchstens 20, vorzugsweise höchstens 18, aufweist.

10. Das Verfahren nach einem der vorhergehenden Ansprüche, **dadurch gekennzeichnet, dass** das Lösungsmittel zusätzlich Wasser enthält, das zur Aufarbeitung, d.h. nach erfolgter Reaktion, zugegeben wird.

11. Das Verfahren nach einem der vorhergehenden Ansprüche, wobei das Lösungsmittel ein unpolares aprotisches Lösungsmittel ist, ausgewählt aus n-Pentan, n-Hexan, Cyclohexan, Methylcyclohexan, Toluol, Benzol, Xylol, Tetrahydrofuran und 1,4-Dioxan; vorzugsweise Toluol oder Tetrahydrofuran; besonders bevorzugt Tetrahydrofuran; wobei das Lösungsmittel vorzugsweise zusätzlich Wasser enthält.

12. Das Verfahren nach einem der vorhergehenden Ansprüche, **dadurch gekennzeichnet, dass** es nicht umfasst:
- eine Kupfer-katalysierte Aminierungsreaktion;
- eine Palladium-katalysierte Aminierungsreaktion; und
- eine Aminierungsreaktion unter Beteiligung einer Organosiliciumbase.

13. Das Verfahren nach einem der vorhergehenden Ansprüche, wobei die in Schritt (b) erhaltene Zusammensetzung nicht umfasst:
- Kupfer(I)-iodid, vorzugsweise kein Kupfersalz;
- Palladium(II)-acetat, [(2-Di-cyclohexylphosphino-3,6-dimethoxy-2',4',6'-triisopropyl-1,1'-Biphenyl)-2-(2'-amino-1,1'-biphenyl)]palladium(II)methansulfonat und Tris(dibenzyliden-aceton)dipalladium(0); vorzugsweise keine Palladiumverbindung;
- 2,2'-Bis(diphenylphosphino)-1,1'-binaphthyl) (BINAP), vorzugsweise kein phosphororganischer Ligand;
- Cäsiumcarbonat, vorzugsweise keine Cäsiumbase; und/oder
- Lithiumhexamethyldisilazid, Natriumhexamethyldisilazid und Kaliumhexamethyldisilazid; vorzugsweise keine Hexamethyldisilazid-Base, besonders bevorzugt keine OrganosiliciumBase.

14. Ein Verfahren zur Herstellung von Ribociclib 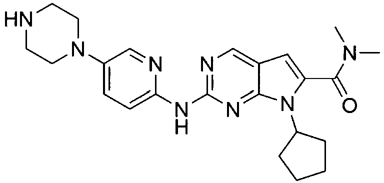 oder eines Säureadditionssalzes davon, wobei das Verfahren ein Verfahren nach einem der vorangehenden Ansprüche und den zusätzlichen Schritt umfaßt:
(g) Entblocken der 1N-Aminogruppe des Piperazinanteils des 4-[6-[[7-Cyclopentyl-6-(dime-thylcarbamoyl)pyrrolo[2,3-d]pyrimidin-2-yl]amino]-3-pyridyl]piperazin-1-carboxy-lats der Formel (I) 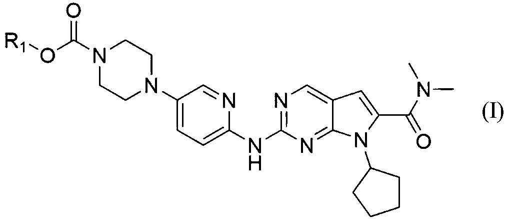 wodurch Ribociclib oder ein Säureadditionssalz von Ribociclib erhalten wird.

15. Das Verfahren zur Herstellung eines physiologisch verträglichen Salzes von Ribociclib, umfassend ein Verfahren nach Anspruch 14 und die zusätzlichen Schritte:
(h) Bereitstellen einer Zusammensetzung, die Ribociclib, eine Säure, ein Lösungsmittel A und gegebenenfalls ein Lösungsmittel B umfasst; wobei das Lösungsmittel A vorzugsweise aus Alkoholen, Ketonen, Estern und Nitrilen ausgewählt ist; und/oder das Lösungsmittel B vorzugsweise aus Alkanen, Ethern, Estern, Alkoholen und Wasser ausgewählt ist;
(i) Ermöglichen, daß das Ribociclib und die Säure ein physiologisch akzeptables Salz von Ribociclib bilden, wodurch eine Produktzusammensetzung bereitgestellt wird; und
(j) gegebenenfalls die Gewinnung zumindest des größten Teils des physiologisch akzeptablen Salzes von Ribociclib aus der Produktzusammensetzung.

## Revendications

1. Procédé de préparation d'un 4-[6-[[7-cyclopentyl-6-(diméthylcarbamoyl)pyrrolo[2,3-d]pyrimidin-2-yl]amino]-3-pyridyl]pipérazine-1-carboxylate de formule (I) 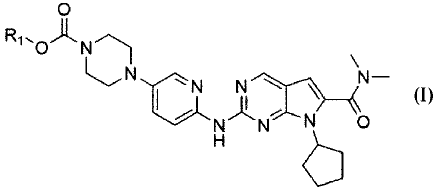 dans lequel R1 représente -alkyle en C₁₋₁₂, -aryle en C₁₋₁₂ ou - alkyl en C₁₋₁₂-aryle en C₁₋₁₆ ;
ledit procédé comprenant les étapes :
(a) de fourniture d'une composition comprenant
(i) un 4-(6-aminopyridin-3-yl)pipérazine-1-carboxylate de formule (III) 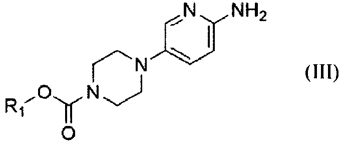 dans lequel R1 représente -alkyle en C₁₋₁₂, -aryle en C₁₋₁₂ ou - alkyl en C₁₋₁₂-aryle en C₁₋₁₆ ;
(ii) un solvant qui est choisi dans le groupe constitué du n-pentane, du n-hexane, du cyclohexane, du méthylcyclohexane, du toluène, du benzène, du xylène, du dichlorométhane, du tétrahydrofurane et du 1,4-dioxane ; et
(iii) une base ;
- dans lequel l'acide conjugué de la base a une valeur de pKa d'au plus 25 ; et
- dans lequel la base a pour formule générale (IV) 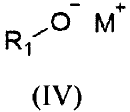 dans lequel M est choisi parmi Li, Na et K ; et R₁ dans la formule générale (IV) est choisi dans le groupe constitué du méthyle, de l'éthyle, du -tert-butyle, du -benzyle, du - méthylène-fluorényle et de l'-allyle ;
(b) d'ajout à la composition fournie à l'étape (a) d'un 7-cyclopentyl-N,N-diméthyl-7H-pyrrolo[2,3-d]pyrimidine-6-carboxamide de formule (II) 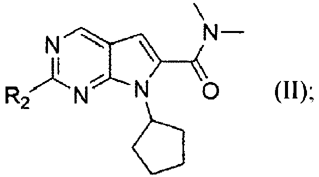 dans lequel R2 représente un groupe partant ;
(c) de réaction de la composition obtenue à l'étape (b) afin d'obtenir une composition comprenant le 4-[6-[[7-cyclopentyl-6-(diméthylcarbamoyl)pyrrolo[2,3-d]pyrimidin-2-yl]amino]-3-pyridyl]pipérazine-1-carboxylate de formule (I) ;
(d) éventuellement, d'ajout d'une quantité supplémentaire de base ; de préférence de la même base que celle qui était également contenue dans la composition fournie à l'étape (a) ;
(e) éventuellement, d'ajout de l'eau à la composition obtenue à l'étape (c) ou (d) ; et
(f) éventuellement, de récupération du 4-[6-[[7-cyclopentyl-6-(diméthylcarbamoyl)pyrrolo[2,3-d]pyrimidin-2-yl]amino]-3-pyridyl]pipérazine-1-carboxylate de formule (I) à partir de la composition obtenue à l'étape (c), (d) ou (e).

2. Procédé selon la revendication 1, dans lequel le rapport stœchiométrique (mole:mole) du 4-(6-aminopyridin-3-yl)pipérazine-1-carboxylate de formule (III) au 7-cyclopentyl-N,N-diméthyl-7H-pyrrolo[2,3-d]pyrimidine-6-carboxamide de formule (II) est d'au moins 0,9, de préférence d'au moins 1,0, plus particulièrement d'au moins 1,05, encore plus particulièrement d'au moins 1,1, et tout particulièrement d'au moins 1,2.

3. Procédé selon la revendication 1 ou 2, dans lequel le rapport stœchiométrique (mole:mole) du 4-(6-aminopyridin-3-yl)pipérazine-1-carboxylate de formule (III) au 7-cyclopentyl-N,N-diméthyl-7H-pyrrolo[2,3-d]pyrimidine-6-carboxamide de formule (II) est d'au plus 2,0, de préférence d'au plus 1,9, plus particulièrement d'au plus 1,8, encore plus particulièrement d'au plus 1,7, plus particulièrement encore d'au plus 1,6, plus particulièrement encore d'au plus 1,5 et tout particulièrement d'au plus 1,4.

4. Procédé selon l'une quelconque des revendications précédentes, dans lequel le rapport stœchiométrique (mole:mole) du 4-(6-aminopyridin-3-yl)pipérazine-1-carboxylate de formule (III) au 7-cyclopentyl-N,N-diméthyl-7H-pyrrolo[2,3-d]pyrimidine-6-carboxamide de formule (II) est dans la plage de 1,20±0,20:1,00, plus particulièrement de 1,20±0,15:1,00, encore plus particulièrement de 1,20±0,10:1,00, et tout particulièrement de 1,20±0,05:1,00.

5. Procédé selon l'une quelconque des revendications précédentes, dans lequel le rapport stœchiométrique (mole:mole) du 4-(6-aminopyridin-3-yl)pipérazine-1-carboxylate de formule (III) au 7-cyclopentyl-N,N-diméthyl-7H-pyrrolo[2,3-d]pyrimidine-6-carboxamide de formule (II) est dans la plage de 1,05±0,05:1,00, plus particulièrement de 1,05±0,04:1,00, encore plus particulièrement de 1,05±0,03:1,00, et tout particulièrement de 1,05±0,02:1,00.

6. Procédé selon l'une quelconque des revendications précédentes, dans lequel la réaction du 4-(6-aminopyridin-3-yl)pipérazine-1-carboxylate de formule (III) et du 7-cyclopentyl-N,N-diméthyl-7H-pyrrolo[2,3-d]pyrimidine-6-carboxamide de formule (II) à l'étape (c) ou (d) se déroule via un mécanisme de substitution nucléophile aromatique (S_{N}Ar).

7. Procédé selon l'une quelconque des revendications précédentes, dans lequel le rapport stœchiométrique (mole:mole) de la base par rapport au 7-cyclopentyl-N,N-diméthyl-7H-pyrrolo[2,3-d]pyrimidine-6-carboxamide de formule (II) est dans la plage de 2,3±2,1:1,0, de préférence de 2,3±1,8:1,0, plus particulièrement de 2,3±1,5:1,0, encore plus particulièrement de 2,3±1,2:1,0, plus particulièrement encore de 2,3±0,9:1,0, encore plus particulièrement de 2,3±0,6:1,0, tout particulièrement de 2,3±0,3:1,0, et en particulier de 2,3±0,2:1,0.

8. Procédé selon l'une quelconque des revendications précédentes, dans lequel
- la base est le tert-butanolate de potassium ; et/ou
- R1 dans la formule générale (III) est choisi dans le groupe constitué du -méthyle, de l'-éthyle, du -tert-butyle, du - benzyle, du -méthylènefluorényle et de l'-allyle ; de préférence R1 dans la formule générale (III) est le -tert-butyle ; et/ou
- R2 dans la formule générale (II) est choisi dans le groupe constitué de -Cl, -Br, -I, -O-S(=O)₂-CH₃, -O-S(=O)₂-CF₃, et -OS(=O)₂-(C₆H₄)-CH₃ ; de préférence R2 dans la formule générale (II) est -Cl.

9. Procédé selon l'une quelconque des revendications précédentes, dans lequel l'acide conjugué de la base a une valeur de pKa d'au plus 20, de préférence d'au plus 18.

10. Procédé selon l'une quelconque des revendications précédentes, dans lequel le solvant contient de plus de l'eau ajoutée pour le traitement, c'est-à-dire après que la réaction a eu lieu.

11. Procédé selon l'une quelconque des revendications précédentes, dans lequel le solvant est un solvant aprotique non polaire choisi parmi le n-pentane, le n-hexane, le cyclohexane, le méthylcyclohexane, le toluène, le benzène, le xylène, le tétrahydrofurane et le 1,4-dioxane ; de préférence le toluène ou le tétrahydrofurane ; plus particulièrement le tétrahydrofurane ; de préférence dans lequel le solvant contient de plus de l'eau.

12. Procédé selon l'une quelconque des revendications précédentes, qui n'implique pas
- une réaction d'amination catalysée par le cuivre ;
- une réaction d'amination catalysée par le palladium ; et
- une réaction d'amination impliquant une base organosiliciée.

13. Procédé selon l'une quelconque des revendications précédentes, dans lequel la composition obtenue à l'étape (b) ne comprend pas
- d'iodure de cuivre (I), de préférence aucun sel de cuivre ;
- d'acétate de palladium (II), de méthanesulfonate de [(2-di-cyclohexylphosphino-3,6-diméthoxy-2',4',6'-triisopropyl-1,1'-biphényl)-2-(2'-amino-1,1'-biphényl)]palladium (II) et de tris (dibenzylidèneacétone)dipalladium(0) ; de préférence aucun composé de palladium ;
- de 2, 2'-bis(diphénylphosphino)-1,1'-binaphtyle) (BINAP), de préférence aucun ligand organophosphoré ;
- de carbonate de césium, de préférence aucune base de césium ; et/ou
- d'hexaméthyldisilazide de lithium, d'hexaméthyldisilazide de sodium et d'hexaméthyldisilazide de potassium ; de préférence aucune base d'hexaméthyldisilazide, et plus particulièrement aucune base organosiliciée.

14. Procédé de préparation du Ribociclib 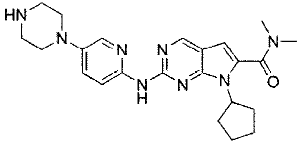 ou un sel d'addition d'acide de celui-ci, le procédé comprenant un procédé selon l'une quelconque des revendications précédentes et l'étape supplémentaire de :
(g) déblocage du goupre 1N-amino du groupement pipérazine du 4-[6-[[7-cyclopentyl-6-(diméthylcarbamoyl)pyrrolo[2,3-d]pyrimidin-2-yl]amino]-3-pyridyl]pipérazine-1-carboxylate de formule (I) 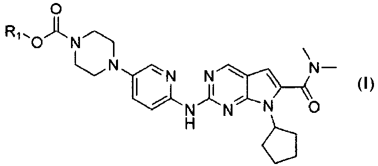 donnant ainsi du ribociclib ou un sel d'addition acide de Ribociclib.

15. Procédé de préparation d'un sel physiologiquement acceptable de ribociclib comprenant un procédé selon la revendication 14 et les étapes supplémentaires de :
(h) fourniture d'une composition comprenant du Ribociclib, un acide, un solvant A et éventuellement un solvant B ; dans lequel le solvant A est de préférence choisi parmi des alcools, des cétones, des esters et des nitriles ; et/ou le solvant B est de préférence choisi parmi des alcanes, des éthers, des esters, des alcools et de l'eau ;
(i) formation, à partir du Ribociclib et de l'acide, d'un sel de Ribociclib physiologiquement acceptable, donnant ainsi une composition de produit ; et
(j) éventuellement, récupération d'au moins la majorité du sel de Ribociclib physiologiquement acceptable à partir de la composition de produit.
